# EUROPEAN PATENT SPECIFICATION

(11) **EP 3 358 487 B1**
(45) Date of publication and mention of the grant of the patent: **19.06.2019**
(21) Application number: 16873184.2
(22) Date of filing: 05.07.2016
(51) Int. Cl.: A61B 5/107, A61B 5/00, A61B 5/1171, G06F 3/01, G01N 3/08, G01L 5/00, G06F 21/32, G06Q 20/40, G02B 27/01

(54) **METHOD FOR AUTHENTICATING USER OF HEAD MOUNTED DISPLAY DEVICE AND HEAD MOUNTED DISPLAY DEVICE**
VERFAHREN ZUR AUTHENTIFIZIERUNG DES BENUTZERS EINER KOPFMONTIERTEN ANZEIGEVORRICHTUNG UND AM KOPF MONTIERTE ANZEIGEVORRICHTUNG
PROCÉDÉ D'AUTHENTIFICATION D'UTILISATEUR DE DISPOSITIF DE VISIOCASQUE ET DISPOSITIF DE VISIOCASQUE

(30) Priority: 10.12.2015 KR 20150176055; 25.05.2016 KR 20160064233
(43) Date of publication of application: 08.08.2018
(73) Proprietor: Samsung Electronics Co., Ltd., Suwon-si, Gyeonggi-do 16677 (KR)
(72) Inventor: YAKISHYN, Yevhenii, Kyiv 02099 (UA); SHCHUR, Oleksandr, Kyiv 01021 (UA); KIM, Sun-kyung, Busan 49516 (KR); FERLIOVSKA, Gelena, Kiev 03188 (UA); ALIEKSIEIEV, Mykola, Kyiv 03062 (UA)
(74) Representative: HGF Limited
(86) International application number: PCT/KR2016/007218
(87) International publication number: WO 2017/099318

(56) References cited:
- WO-A2-2014/021602
- KR-A- 20150 059 085
- KR-A- 20150 106 229
- US-A1- 2012 206 322
- US-A1- 2014 071 041
- US-B1- 8 963 806
- US-B1- 8 963 806

## Description

### [Technical Field]

The present disclosure relates to wearable display devices, and more particularly, to head mounted display (HMD) devices.

### [Background Art]

To meet the lightweight and miniaturization trends of digital devices, various wearable devices are under development. A head mounted display (HMD) device, which is a kind of a wearable device, is a digital device worn by a user on the head to receive multimedia content or experience virtual reality (VR) or augmented reality (AR). The HMD device may have various forms to be worn on the head such as glasses or a part of a helmet.

When the HMD device is used, authenticating a use authority of a user who uses the HMD device is an important issue for protection of personal information, security, content providing, etc. A method of authenticating an HMD device may be implemented in various ways. For example, a method of authenticating the HMD device may be implemented via an iris authentication algorithm performed by an iris recognition scanner to recognize an iris of a user who wears the HMD device or an authentication algorithm for recognition of a user's face.

US-8963806-B1 discloses a head-mountable device configured to authenticate a wearer is disclosed. The head-mountable device can receive an indication of an eye gesture from at least one proximity sensor in the head-mountable device configured to generate sensor data indicative of light reflected from an eye area. The head mountable device can capture biometric information indicative of one or more biometric identifiers of a wearer of the head-mountable device responsive to receiving the indication of the eye gesture. The head-mountable device can authenticate the wearer of the head-mountable device based on a comparison of the captured biometric information and a stored biometric profile.

### [Disclosure of Invention]

### [Solution to Problem]

Provided are methods of authenticating a use authority of a user who wears a head mounted display (HMD) device and an HMD device performing the method.

Provided are methods of identifying a specific user from among a plurality of users who use an HMD device and an HMD device performing the method.

### [Advantageous Effects of Invention]

An embodiment of present disclosure provides a convenient, accurate, and reliable biometric authentication method by obtaining a curvature curve for a head shape of a user who wears the HMD device via the plurality of sensors included in the HMD device, and authenticating a use authority of the user. Also, the authentication method according to the embodiment does not require an additional iris recognition sensor, face scanner, etc., so that additional costs may be saved and thus the authentication method is advantageous in an aspect of costs.

### [Brief Description of Drawings]

FIG. 1A is a view for explaining a geometric structure of a human skull, and FIG. 1B is a diagram illustrating a shape of a human skull;
FIGS. 2A and 2B are views for explaining a method of authenticating, at a head mounted display (HMD) device, a use authority of a user, and an HMD device according to an embodiment;
FIG. 3 is a block diagram for explaining components of an HMD device according to an embodiment;
FIG. 4 is a flowchart for explaining a method of authenticating, at an HMD device, a use authority of a user who wears the HMD device according to an embodiment;
FIGS. 5A to 5C are views for explaining a method of obtaining, at a sensor, a characteristic value of a head shape of a user when the sensor contacts the user's head according to an embodiment;
FIGS. 6A to 6C are views illustrating locations at which a plurality of sensors included in an HMD device and spaced apart from each other contact a user's head according to an embodiment;
FIG. 7 is a view for explaining a method of measuring, at an HMD device, a characteristic value of a head shape of a user according to an embodiment;
FIG. 8 is a view for explaining a method of classifying characteristic values of a head shape of a user measured by an HMD device into a plurality of series according to an embodiment;
FIG. 9 is a view for explaining a method of measuring, at an HMD device, a characteristic value of a head shape of a user according to an embodiment;
FIG. 10 is a view for explaining a method of classifying characteristic values of a head shape of a user measured by an HMD device into a plurality of series according to an embodiment;
FIG. 11 is a view for explaining a method of measuring, at an HMD device, a characteristic value of a head shape of a user according to an embodiment;
FIG. 12 is a view for explaining a method of classifying characteristic values of a head shape of a user measured by an HMD device into a plurality of series according to an embodiment;
FIG. 13 is a flowchart for explaining a method of authenticating a use authority of a user who wears an HMD device via characteristic values of a head shape of a user measured by the HMD device via a plurality of sensors spaced apart from each other according to an embodiment;
FIG. 14 is a view illustrating locations at which a plurality of sensors included in an HMD device and spaced apart from each other contact a user's face according to an embodiment;
FIG. 15 is a view for explaining a method of classifying characteristic values of a face shape of a user measured by an HMD device into a plurality of series according to an embodiment;
FIG. 16A is a perspective view of an HMD device including a conductive sensor configured to measure a characteristic value regarding a user's facial shape according to an embodiment;
FIG. 16B is an enlarged cross-sectional view of a portion of the HMD device illustrated in FIG. 16A;
FIGS. 17A and 17B are graphs of characteristic values regarding a user's facial shape measured by a conductive sensor included in an HMD device according to an embodiment;
FIG. 18A is a perspective view of an HMD device including a lighting element and a photosensitive element configured to measure characteristic values regarding a user's facial shape according to an embodiment;
FIG. 18B is an enlarged cross-sectional view of a portion of the HMD device illustrated in FIG. 18A;
FIGS. 19A and 19B are graphs of characteristic values regarding a user's facial shape detected by a photo resister included in an HMD device according to an embodiment;
FIG. 20A is a perspective view of an HMD device including a conductive tube and an electrode configured to measure characteristic values regarding a user's facial shape according to an embodiment;
FIG. 20B is an enlarged cross-sectional view of a portion of the HMD device illustrated in FIG. 20A;
FIG. 20C is an enlarged perspective view of a conductive rubber tube and an electrode pad illustrated in FIG. 20A;
FIGS. 21A and 21B are views for explaining a method of measuring characteristic values regarding a user's facial shape corresponding to a shape change of a conductive rubber tube in the case where a user does not wear an HMD device and in the case where a user wears an HMD device according to an embodiment;
FIGS. 22A and 22B are graphs of characteristic values regarding a user's facial shape detected by an electrode pad included in an HMD device according to an embodiment.
FIGS. 23A and 23B are graphs illustrating deviation between a characteristic value obtained by measuring a characteristic value of a head shape of a user who wears another HMD device over time and a characteristic value registered in advance according to an embodiment;
FIG. 24 is a flowchart for explaining a method of measuring a characteristic value of a head shape of a user who wears an HMD device over time, and authenticating a use authority of the user;
FIG. 25 is a flowchart for explaining a method of authenticating a use authority of a user by comparing a characteristic value of a head shape of a user who wears an HMD device with a characteristic value stored in advance according to an embodiment;
FIG. 26 is a flowchart for explaining a method of identifying a user who wears an HMD device from among a plurality of users who use the HMD device according to an embodiment;
FIG. 27 is a flowchart for explaining a method of identifying a user by comparing a characteristic value of a head shape of a user who wears an HMD device with characteristic values of a plurality of users registered in advance according to an embodiment; and
FIG. 28 is a block diagram for explaining components of an HMD device according to an embodiment.

### [Best Mode for Carrying out the Invention]

According to an aspect of an embodiment, a head mounted display (HMD) device includes: a plurality of sensors configured to measure a characteristic value of a head shape of a user who wears the HMD device; a memory configured to store a characteristic value registered in advance of a head shape of a user who has an authority to use the HMD device; and a controller configured to authenticate an authority allowing the user who wears the HMD device to use the HMD device based on the measured characteristic value and the characteristic value registered in advance; wherein the sensors of the plurality of sensors are spaced apart around an entire or partial surface of a skull of the user; and wherein the sensors of the plurality of sensors are configured to measure a characteristic value indicating a geometric and structural characteristic of the skull of the user who wears of the HMD device.

According to an aspect of another embodiment, a method of authenticating at a head mounted display (HMD) device including a plurality of sensors a user of the HMD device, the method includes: measuring a characteristic value of a head shape of the user who wears the HMD device; and authenticating a use authority of the user who wears the HMD device based on the measured characteristic value and a characteristic value registered in advance of a head shape of an authenticated user having an authority to use the HMD device; wherein the sensors of the plurality of sensors are spaced apart around an entire or partial surface of a skull of the user; and wherein the measuring of the characteristic value comprises measuring a characteristic value indicating a geometric and structural characteristic of the skull of the user who wears the HMD device.

According to an aspect of another embodiment, a non-transitory computer-readable recording medium having recorded thereon a program for executing the above-described method on a computer is provided.

### [Mode for the Invention]

Embodiments are described below in detail with reference to the accompanying drawings to enable a person of ordinary skill in the art to easily carry out the inventive concept. However, the embodiments may be implemented in various different forms and are not limited to the descriptions herein. For a clear description, portions irrelevant to the description have been omitted and like reference numerals are used for like components throughout the specification.

Throughout the specification, it will be understood that when a component is referred to as being "connected" to another component, it may be "directly connected" to the other component or may be "electrically connected" to the other component with other component disposed therebetween. Also, it will be understood that when a component "comprises" a certain component, it may further comprise another component and does not preclude the presence of another component unless specified otherwise.

Embodiments are described below with reference to the accompanying drawings.

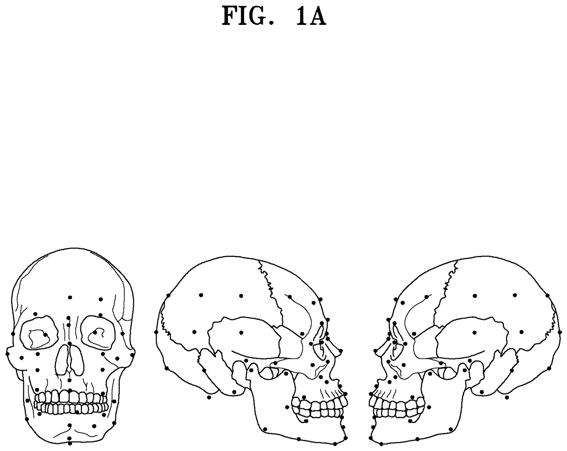

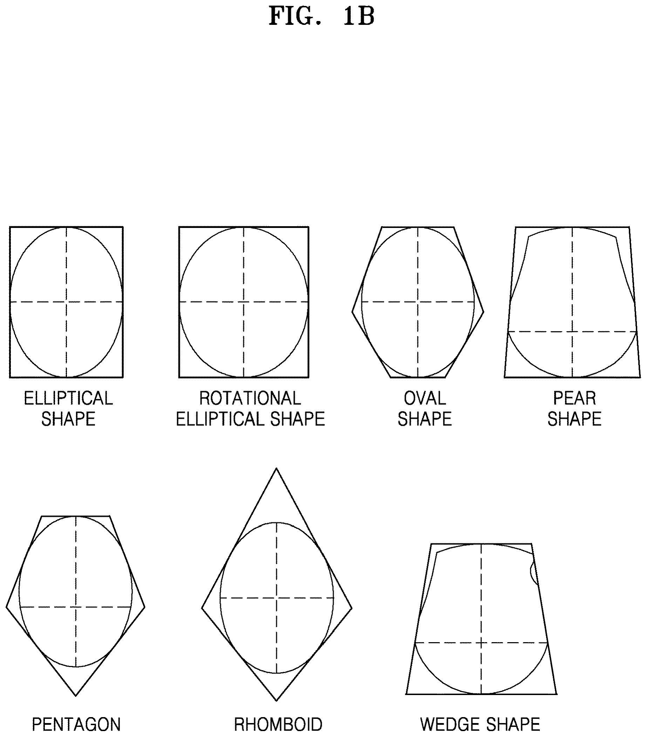

FIG. 1A is a view for explaining a geometric structure of a human skull, and FIG. 1B is a diagram illustrating a shape of a human skull.

Referring to FIG. 1A, characteristic points representing the shape, that is, the geometric structure of a human skull are shown. For example, the shape of the human skull may be specified via the Manchester protocol. Like the fingerprint and an iris, the shape of the human skull has different characteristics for each race and each person. For example, a shape, a size, a curvature, etc. of a skeleton such as a frontal bone, a temporal bone, an occipital bone, a glabella, and a supraorbital foramen of a person include unique characteristics different for each individual.

Referring to FIG. 1B, shapes of human heads may be shown as a diagram and classified for each kind. A head shape, that is, a shape of a human skull may mean at least one of a shape, a size, a skeletal structure, and a curvature of the skull. A human skull may be classified into an ellipsoid, a spheroid, an ovoid, pear-shaped, a pentagonoid, a rhomboid, a sphenoid, etc. However, the shape of a human skull is not limited to the listed shape.

Referring to FIGS. 1A and 1B, a shape of a person's head, that is, a shape of a human skull includes geometric characteristics different for each individual. Therefore, a head mounted display (HMD) device 1000 mounted on a human head to be in physical contact with the human head measures characteristic values of a head shape via sensors, which may be utilized in a method of authenticating a use authority of a user.

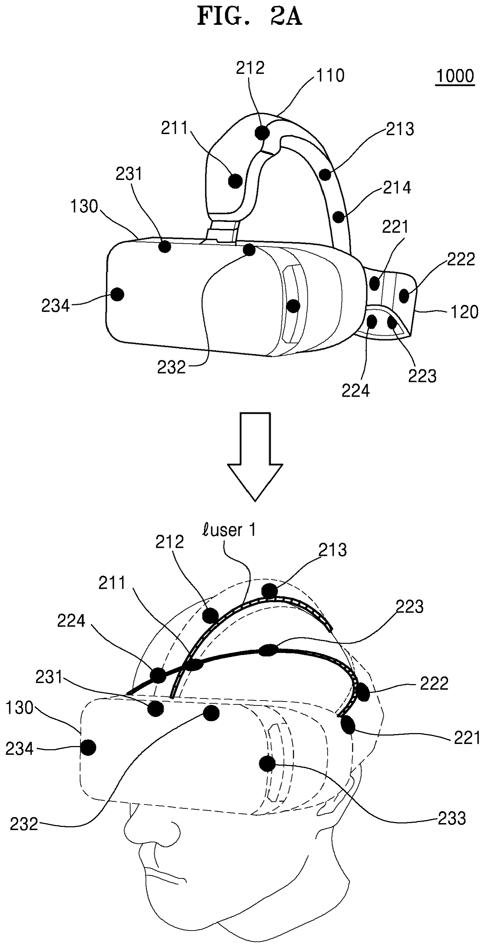

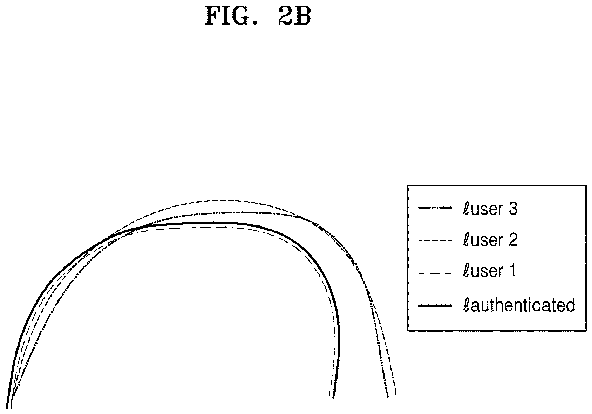

FIGS. 2A and 2B are views for explaining a method of authenticating, at an HMD 1000 device, a use authority of a user, and the HMD 1000 device according to an embodiment. The HMD device 1000 is a display device that can be mounted on a user's head. Specifically, the HMD device 1000, which is a kind of a wearable device, can be any of various digital devices allowing a user to wear it on the head and receive multimedia content or experience virtual reality (VR) or augmented reality (AR).

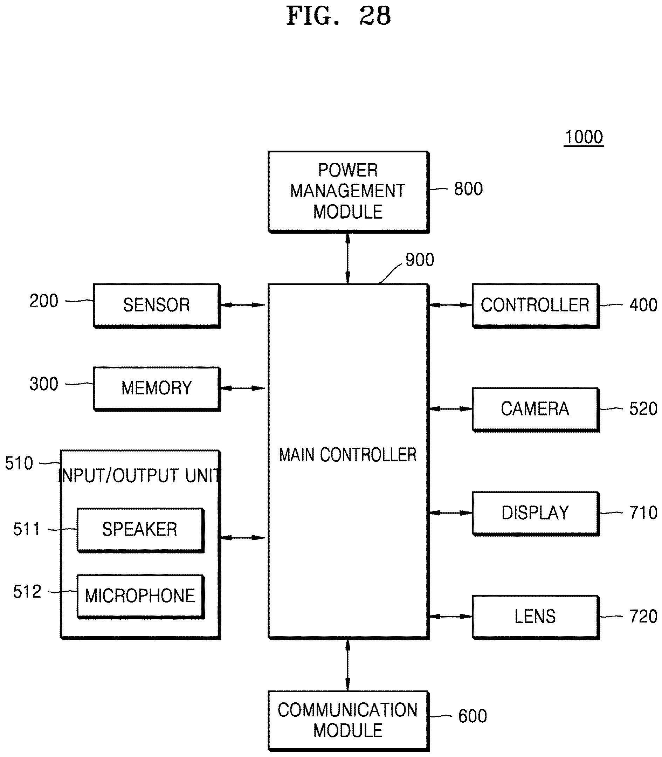

Referring to FIG. 2A, with regard to an appearance, the HMD device 1000 may include a mounting belt 110 surrounding and supporting a partial region on the skull of a user, a strap band 120 surrounding and supporting a frontal bone, a temporal bone, and an occipital bone of the user, and a mask 130 covering a partial region of the user's face. In an embodiment, the HMD device 1000 may further include other components such as a display 710 (see FIG. 28) and a lens 720 (see FIG. 28). These components are described below with reference to FIG. 28.

The HMD device 1000 may further include a plurality of sensors 211 to 214 spaced apart from each other inside the mounting belt 110, a plurality of sensors 221 to 224 spaced apart from each other inside the strap band 120, and a plurality of sensors 231 to 234 spaced apart from each other inside the mask 130. In an embodiment, the plurality of sensors 211 to 234 may measure characteristic values of a head shape of a user who wears the HMD device 1000. The head shape of the user may indicate geometrical and structural characteristics including a size, a shape, a skeletal structure of a skull of the user, a curvature of the skull, or a size, a shape, a curvature, an arrangement of eyes, a nose, and a mouth of a face of the user.

The characteristic values of the head shape of the user may mean physical and geometric parameters of a geometric and structural characteristic for the head shape of the user. For example, the characteristic values of the head shape of the user may include at least one of geometric parameters including a tension value, a pressure value, a stress value, and a strain value of a skull of the user or a face of the user measured by using the plurality of sensors 211 to 234.

In an embodiment, the plurality of sensors 211 to 234 may include at least one of a tension sensor, a pressure sensor, a stress sensor, and a strain sensor, but are not limited to the listed sensors. In an embodiment, the HMD device 1000 may measure, by using the plurality of sensors 211 to 214, a curvature of a skull of a first user who wears the HMD device 1000, and compare the measured a curvature with a curvature of a skull of an authenticated user (see FIG. 2B) registered in advance, thereby authenticating a use authority of the user.

In another embodiment, the HMD device 1000 may measure, by using the plurality of sensors 221 to 224, a curvature of a temporal bone and an occipital bone of a user who wears the HMD device 1000, and compare the measured curvature with a curvature of a temporal bone and an occipital bone of a user registered in advance, thereby authenticating a use authority of the user.

The HMD device 1000 may measure a characteristic of a head shape of a user by using physical and mechanical measurement methods via physical contact with a portion of a body, a head of a user, and compare the measured characteristic with a characteristic for a head shape of a user registered in advance, thereby authenticating a use authority of the user.

Specifically, the HMD device 1000 may measure a characteristic value of a head shape of a user who wears the HMD device 1000 by using the plurality of sensors 211 to 234 included inside the HMD device 1000, thereby authenticating a use authority of the user who uses the HMD device 1000.

In an embodiment, the HMD device 1000 may measure characteristic values of a head shape of a plurality of users who use the HMD device 1000 by using the plurality of sensors 211 to 234 included inside the HMD device 1000, register the characteristic values, measure a characteristic value of a head shape of a first user who wears the HMD device 1000, and compare the measured characteristic value with the plurality of characteristic values registered in advance, thereby authenticating the first user.

Referring to FIG. 2B, curvature curves I_{user 1}, I_{user 2}, and I_{user 3} of a skull of a plurality of users measured by using the plurality of sensors 211 to 234, and a curvature curve I_{authenticated} of a skull of an authenticated user registered in advance are illustrated. In an embodiment, the HMD device 1000 may obtain the curvature curves I_{user 1}, I_{user 2}, and I_{user 3} of the plurality of users by measuring a curvature of a partial or entire skull of the plurality of users who wear the HMD device 1000 by using the plurality of sensors 211 to 214 spaced apart from each other inside the mounting belt 110 (see FIG. 2A).

The HMD device 1000 may compare the curvature curves I_{user 1}, I_{user 2}, and I_{user 3} of the plurality of users with the curvature curve I_{authenticated} of the user registered in advance who has a use authority to use the HMD device 100, thereby determining an authenticated user from among the plurality of users. In an embodiment, the HMD device 1000 may obtain not only a curvature curve of a skull but also curvature curves of a temporal bone and an occipital bone, and compare curvature curves of a temporal bone and an occipital bone registered in advance with the obtained curvature curves, thereby authenticating a use authority of a user who wears the HMD device 1000.

Recently, in using the HMD device 1000, authentication of a use authority rises as an important issue. Particularly, authenticating a use authority of a user who uses the HMD device 1000 is important for protection and security of personal information. Particularly, in the case of the HMD device 1000 used in common by family, teenagers may receive content for an adult by using the HMD device 1000. Therefore, authentication of a use authority is surely required.

An embodiment illustrated in FIGS. 2A and 2B may provide a convenient, accurate, and reliable biometric authentication method by obtaining a curvature curve for a head shape of a user who wears the HMD device 1000 via the plurality of sensors 211 to 234 included in the HMD device 1000, and authenticating a use authority of the user. Also, the authentication method according to an embodiment does not require an additional iris recognition sensor, face scanner, etc., so that additional costs may be saved and thus the authentication method is advantageous in an aspect of costs.

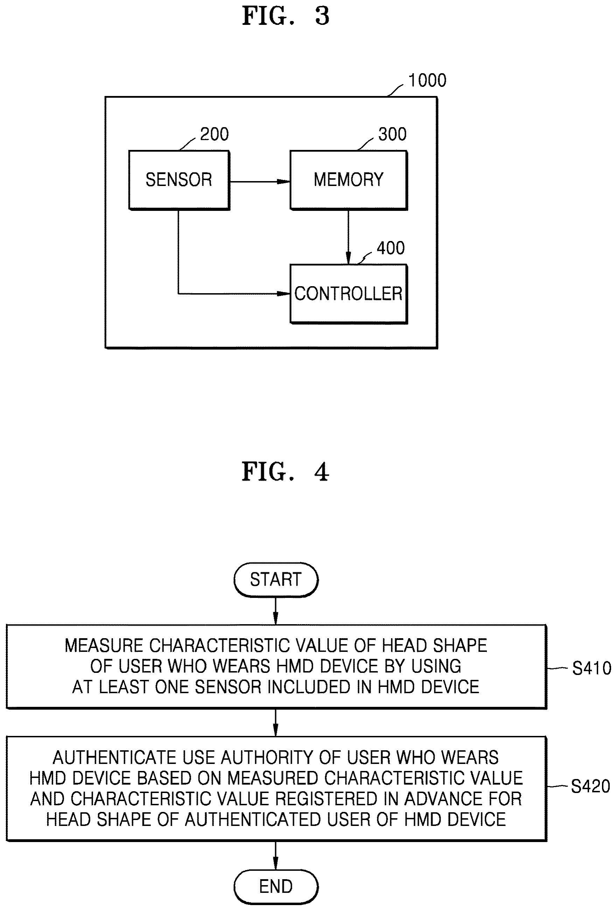

FIG. 3 is a block diagram for explaining components of an HMD device 1000 according to an embodiment.

Referring to FIG. 3, the HMD device 1000 may include a sensor 200, a memory 300, and a controller 400. However, the HMD device 1000 does not include only the listed components, and may further include a support member including the mounting belt 110, the strap band 120, and the mask 130 (see FIG. 2A).

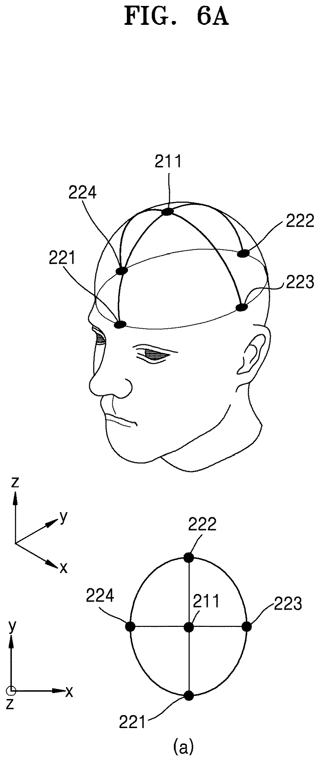

The sensor 200 may measure a characteristic value of a head shape of a user who wears the HMD device 1000. The sensor 200 may be disposed in one of the mounting belt 110, the strap band 120, and the mask 130 (see FIG. 2A). In the case where a user wears the HMD device 1000 on his head, the sensor 200 may physically and mechanically contact the user's head. Arrangement of the sensor 200 is described below in relevant description portions with reference to FIGS. 6A to 6C, 7, 9, 11, and 14.

In an embodiment, the sensor 200 may measure at least one of a tension value, a pressure value, a stress value, and a strain value of an entire or partial region of a skull or a face of a user who wears the HMD device 1000. The sensor 200 may include one sensor or a plurality of sensors. In an embodiment, the sensor 200 may include at least one of a tension sensor, a pressure sensor, a stress sensor, a strain sensor, a conductivity sensor, and a lighting sensor.

The memory 300 may store a characteristic value registered in advance of a head structure of an authenticated user having an authority to use the HMD device 1000. In an embodiment, the memory 300 may classify characteristic values registered in advance of each series of characteristic values obtained from each of the plurality of sensors 200 spaced apart from each other and store the same, or store characteristic values successively obtained by the sensor 200 over time in time series.

In an embodiment, the memory may store characteristic values of head shapes of a plurality of users who use the HMD device 1000 measured by the sensor 200. The memory 300 may classify and store a plurality of characteristic values obtained by each of the sensors 200 spaced apart from each other for each series, or respectively store characteristic values successively obtained by the sensors 200 over time in time series.

The memory 300 may include at least one of an internal memory and an external memory.

The internal memory may include, for example, at least one of a volatile memory (for example, dynamic RAM (DRAM), static RAM (SRAM), synchronous dynamic RAM (SDRAM), etc.), a non-volatile memory (for example, one time programmable ROM (OTPROM), programmable ROM (PROM), erasable and programmable ROM (EPROM), electrically erasable and programmable ROM (EEPROM), mask ROM, flash ROM, etc.), a hard disk drive (HDD), and a solid state drive (SSD).

The external memory may include, for example, at least one of compact flash (CF), secure digital (SD), micro-SD, mini-SD, extreme digital (xD), and a memory stick.

The controller 400 may compare a characteristic value of a head shape of a user measured by the sensor 200 with a characteristic value registered in advance and stored in the memory 300, thereby authenticating a use authority of the user. In an embodiment, the controller 400 may compare a series of characteristic values of a head shape of a user obtained by the sensor 200 with a series of characteristic values registered in advance, thereby authenticating a use authority of the user.

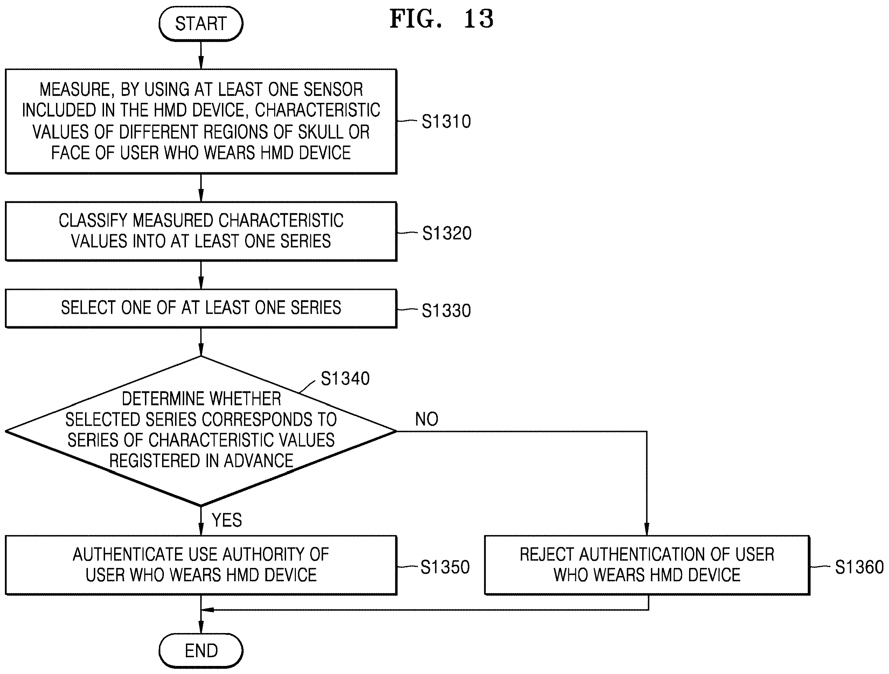

In another embodiment, the controller 400 may compare a characteristic value of a head shape of a user successively measured over time by the sensor 200 with a characteristic value stored in advance in the memory 300, thereby authenticating a use authority of the user. A method of authenticating, at the controller 400, a user who wears the HMD device 1000 is described with reference to FIGS. 13, 23A to 25.

In another embodiment, the controller 400 may compare a characteristic value of a head shape of a specific user, for example, a first user who wears the HMD device 1000 with a plurality of characteristic values stored in the memory 300, thereby identifying the first user. In an embodiment, the controller 400 may calculate a difference value between the characteristic value of the head shape of the first user and each of the plurality of characteristic values stored in the memory 300, and determine a characteristic value having a smallest calculated difference value from among the plurality of characteristic values as a characteristic value of the first user.

The controller 400 may include a central processing unit (CPU) including an operation circuit and a logic circuit. In an embodiment, the controller 400 may include an authentication module including at least one of a CPU, RAM, ROM, GPU, and a BUS. In an embodiment, the controller 400 may be implemented as an application processor (AP). In an embodiment, the controller 400 may be implemented by using a hardware component such as an FPGA or an ASIC. However, the controller 400 is not limited thereto and may include software components, components such as object-oriented software components, class components, and task components, processes, functions, attributes, procedures, sub-routines, segments of a program code, drivers, firmware, a micro code, a circuit, data, a database, data structures, tables, arrays, and variables.

FIG. 4 is a flowchart for explaining a method of authenticating, at an HMD device, a use authority of a user who wears the HMD device according to an embodiment.

In operation S410, the HMD device measures a characteristic value of a head shape of a user who wears the HMD device. In an embodiment, the HMD device may measure at least one of geometric parameters including a tension value, a pressure value, a stress value, and a strain value of a skull or a face of a user who wears the HMD device.

In operation S420, the HMD device authenticates a use authority of the user who wears the HMD device based on the characteristic value measured in operation S410 and a characteristic value registered in advance of a head shape of an authenticated user of the HMD device. In an embodiment, the characteristic value registered in advance of the head shape of the authenticated user of the HMD device may be stored in the HMD device. In an embodiment, the characteristic value registered in advance of the head shape of the authenticated user of the HMD device 1000 may be stored in the memory 300.

The HMD device may compare a series of obtained characteristic values of a head shape of a user with a series of stored characteristic values registered in advance, or compare a characteristic value of a head shape of a user successively measured over time with a characteristic value registered in advance in the HMD device, thereby authenticating a use authority of the user.

In an embodiment, the HMD device authenticates a use authority of a user who wears the HMD device based on the above-described comparison result. When a measured characteristic value of a head shape of a user corresponds to a characteristic value registered in advance, the HMD device may authenticate a use authority of the user.

Also, when a difference between a measured characteristic value of a head shape of a user and a characteristic value registered in advance stored in advance in the HMD device is less than a threshold value set in advance, the HMD device may authenticate a use authority of the user. When the use authority of the user who wears the HMD device is authenticated, the HMD device may perform an operation of displaying multimedia content or providing virtual reality or augmented reality.

In contrast, when a difference between the measured characteristic value and the characteristic value registered in advance stored in advance in the HMD device is equal to or greater than the threshold value set in advance, the HMD device may reject authentication of the use authority of the user. When the authentication of the use authority of the user who wears the HMD device is rejected, the HMD device may not perform a separate operation and power may be turned off.

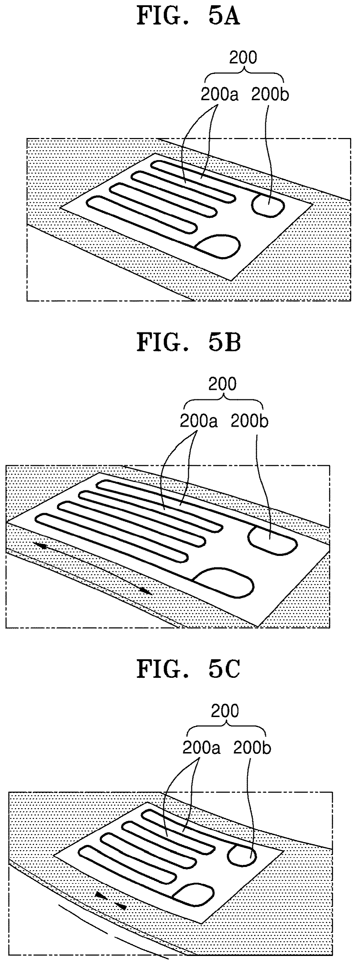

FIGS. 5A to 5C are views for explaining a method of obtaining a characteristic value of a head shape of a user in the case where the sensor 200 contacts the user's head according to an embodiment. In an embodiment illustrated in FIGS. 5A to 5C, the sensor 200 may contact one region of the user's head, and detect an electric resistance value changing depending on a contacted form. In an embodiment, the sensor 200 may include a strain sensor or a strain gauge. A strain measuring unit is only an example of the sensor 200, and the sensor 200 may include at least one of a tension sensor, a pressure sensor, a stress sensor, a conductivity sensor, and a lighting sensor, and is not limited to the listed examples.

Referring to FIG. 5A, the sensor 200 may include a resistance pattern line 200a and a resistance detection terminal 200b. The resistance pattern line 200a may be arranged in zigzags in a mutually parallel direction. In the case where the HMD device 1000 is mounted on a user's head, the resistance pattern line 200a may change its length depending on the user's head shape. The resistance detection terminal 200b may convert a length change of the resistance pattern line 200a with respect to a unit length of the resistance pattern line 200a into an electric resistance value, and detect a change of the converted electric resistance value. In an embodiment, the resistance detection terminal 200b may detect a change of an electric resistance value by using a wheat-stone bridge.

Referring to FIGS. 5B and 5C, the sensor 200 may detect a change of an electric resistance value by a user's head shape. In an embodiment illustrated in FIG. 5B, in the case where a user's head shape is convex in an upper direction, the resistance pattern line 200a receives tensile force, and the length of the resistance pattern line 200a may increase and the width of the resistance pattern line 200a may be reduced. In this case, the resistance detection terminal 200b may detect an increase of an electric resistance. In an embodiment illustrated in FIG. 5C, unlike FIG. 5B, a user's head shape is concave in a lower direction and thus the resistance pattern line 200a contracts, and the length of the resistance pattern line 200a may be reduced and the width of the resistance pattern line 200a may be increased. In this case, the resistance detection terminal 200b may detect a decrease of an electric resistance.

In an embodiment, characteristic values of a head shape of a user who wears the HMD device 1000, that is, a resistance value, a pressure value, a stress change value, etc. may be measured by using the method illustrated in FIGS. 5B and 5C. However, content illustrated in FIGS. 5A to 5C is only an example, and a kind of the sensor 200 and a method of measuring a characteristic value by using the sensor 200 are not limited to the described content.

FIGS. 6A to 6C are a perspective view and a plan view illustrating locations at which a plurality of sensors 211, 221 to 228 spaced apart from each other inside an HMD device 1000 contact a user's head in the case where the HMD device 1000 is mounted on the user's head according to an embodiment.

Referring to FIG. 6A, the plurality of sensors 211, 221 to 224 may be disposed in one region on a skull of the user, or disposed in one region of the frontal bone, the temporal bone, and the occipital bone. Specifically, in the case where the HMD device 1000 is mounted on a user's head, the first sensor 211 disposed inside the mounting belt 110 may be disposed in a partial region on the skull of the user and may contact the skull of the user with the user's skin tissue disposed therebetween. Also, in the case where the HMD device 1000 is mounted on the user's head, the (2-1)-st sensor 221 disposed inside the strap band 120 may be disposed in a first region adjacent to a glabella, the (2-2)-nd sensor 222 may be disposed in a second region on the occipital bone, the (2-3)-rd sensor 223 and the (2-4)-th sensor 224 may be disposed in a third region on the left temporal region and a fourth region on the right temporal region and may contact the user's frontal bone, the temporal bone, and the occipital bone with the user's skin tissue disposed therebetween.

Referring to the plan view illustrated in FIG. 6A, the (2-1)-st sensor 221 and the (2-2)-nd sensor 222 form a virtual straight line in a y-axis direction, the (2-3)-rd sensor 223 and the (2-4)-th sensor 224 may form a virtual straight line in an x-axis direction and may be connected to each other. The first sensor 211 may be disposed at an intersection point of the virtual straight line formed by the (2-1)-st sensor 221 and the (2-2)-nd sensor 222, and the virtual straight line formed by the (2-3)-rd sensor 223 and the (2-4)-th sensor 224. In an embodiment, the first sensor 211, the (2-1)-st sensor 221 to the (2-4)-th sensor 224 may be implemented as at least one of a tension sensor, a pressure sensor, a stress sensor, and a strain sensor.

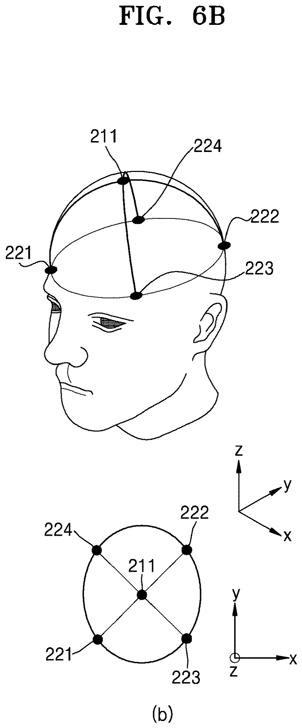

Referring to FIG. 6B, the plurality of sensors 211, 221 to 224 may be disposed in one region on the skull of the user, or in one region of the frontal bone, the temporal bone, and the occipital bone. The arrangement of the plurality of sensors 211, 221 to 224 illustrated in FIG. 6B is an embodiment in which locations of the plurality of sensors 211, 221 to 224 illustrated in FIG. 6A are moved by a predetermined distance clockwise.

Specifically, in the case where the HMD device 1000 is mounted on the user's head, the (2-1)-st sensor 221 disposed inside the strap band 120 may be disposed in a fifth region between a glabella and the right temporal bone, the (2-2)-nd sensor 222 may be disposed in a sixth region between the occipital bone and the left temporal bone, the (2-3)-rd sensor 233 may be disposed in a seventh region between a glabella and the left temporal bone, and the (2-4)-th sensor 224 may be disposed in an eighth region between the temporal bone and the occipital bone, and may contact the user's frontal bone, the temporal bone, and the occipital bone with the user's skin tissue disposed therebetween. The first sensor 211 may be disposed at the same location as that illustrated in FIG. 6A.

Referring to the plan view illustrated in FIG. 6B, the (2-1)-st sensor 221 and the (2-2)-nd sensor 222 form a virtual diagonal line having a predetermined angle between 0° and 90° counterclockwise with respect to the x-axis direction, and the (2-3)-rd sensor 223 and the (2-4)-th sensor 224 form a virtual diagonal line having a predetermined angle between 90° and 180° counterclockwise with respect to the x-axis direction, and may be connected to each other. As described with reference to FIG. 6A, the first sensor 211, the (2-1)-st sensor 221 to the (2-4)-th sensor 224 may be implemented as at least one of a tension sensor, a pressure sensor, a stress sensor, and a strain sensor.

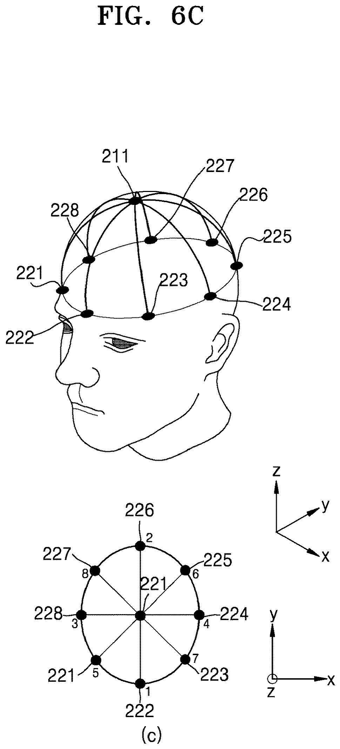

Referring to FIG. 6C, a plurality of sensors 211, 221 to 228 may be disposed in one region on the skull of the user, or disposed in one region of the frontal bone, the temporal bone, and the occipital bone. The arrangement of the plurality of sensors 211, 221 to 228 illustrated in FIG. 6C is an embodiment that combines the locations of the plurality of sensors 211, 221 to 224 illustrated in FIGS. 6A and 6B. Specifically, in the case where the HMD device 1000 is mounted on the user's head, eight sensors in total may be disposed inside the strap band 120, and the (2-1)-st sensor 221 to the (2-8)-th sensor 228 may be disposed on the user's frontal bone, the left temporal bone, the occipital bone, and the right temporal bone counterclockwise with the user's skin tissue disposed therebetween. Referring to the plan view illustrated in FIG. 6C, the (2-1)-st sensor 221 to the (2-8)-th sensor 228 respectively form virtual straight lines in the x-axis direction and the y-axis direction, and form two virtual diagonal lines and may be connected to each other. As described with reference to FIG. 6C, the first sensor 211, the (2-1)-st sensor 221 to the (2-4)-th sensor 224 may be implemented as at least one of a tension sensor, a pressure sensor, a stress sensor, and a strain sensor.

FIGS. 6A to 6C illustrate only an example in which the plurality of sensors 211, 221 to 228 are disposed at specific locations of the user's head in the case where the HMD device 1000 is mounted on the user's head, and do not limit the locations of the plurality of sensors 211, 221 to 228 to the locations illustrated in FIGS. 6A to 6C. Also, the number of the first sensor 211, the (2-1)-st sensor 221 to the (2-8)-th sensor 228 is not limited to the number illustrated in FIGS. 6A to 6C.

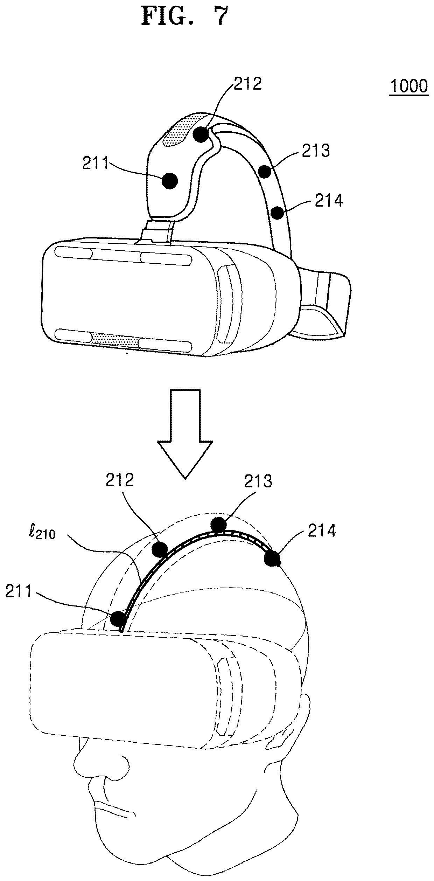

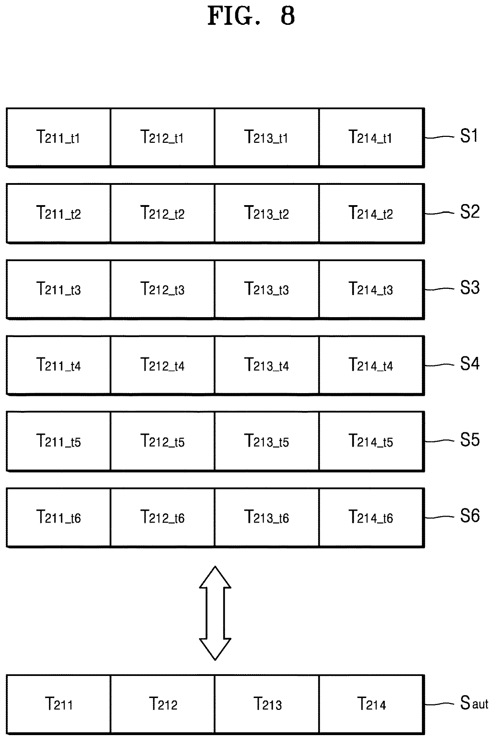

FIG. 7 is a view for explaining a method of measuring, at the HMD device 1000, a characteristic value of an upper surface of the skull of a user according to an embodiment, and FIG. 8 is a view illustrating an embodiment of classifying, at the HMD device 1000, characteristic values obtained by using the method illustrated in FIG. 7 into a plurality of series S1 to S6.

Referring to FIG. 7, the HMD device 1000 may include the mounting belt 110 and a plurality of sensors 211 to 214 spaced apart from each other inside the mounting belt 110. The plurality of sensors 211 to 214 may contact an entire or partial upper surface of the skull of the user with the user's skin tissue disposed therebetween.

In an embodiment, the plurality of sensors 211 to 214 may be tension sensors measuring tension (or tensile force) changed by a shape, a size, or a skeletal structure of an entire or partial upper surface of the skull of the user. However, the plurality of sensors 211 to 214 are not limited thereto and may be at least one of pressure sensors, stress sensors, a strain sensors, conductivity sensors, and lighting sensors measuring pressure values or stress change values changed by a shape, a size, or a skeletal structure of an entire or partial upper surface of the skull of the user.

In an embodiment, the plurality of sensors 211 to 214 may measure tension values changed by an entire or partial upper surface of the skull of the user for predetermined authentication duration that authenticates a use authority of a user who wears the HMD device 1000, and classify measured tension values into a plurality of series and list the classified tension values in a time series.

Referring to FIG. 8, tension values T_{211_t1} to T_{214_t6} by an entire or partial upper surface of the skull of the user obtained by the plurality of sensors 211 to 214 are classified into the plurality of series S1 to S6 and listed. The plurality of series S1 to S6 may be measured for the authentication duration and obtained in a time series. Specifically, the first series S1 may be a set of tension values T_{211_t1} to T_{214_t1} measured at a first time t1 within the authentication duration by using the plurality of sensors 211 to 214. The second series S2 may be a set of tension values T_{211_t2} to T_{214_t2} measured at a second time t2 within the authentication duration by using the plurality of sensors 211 to 214. The third series S3 to the sixth series S6 may be sets of tension values measured at a third time t3 to a sixth time t6, respectively.

The plurality of sensors 211 to 214 may measure a curvature of an entire or partial skull of the user by combining the tension values included in each of the plurality of series S1 to S6, and may obtain a curvature curve I₂₁₀ for the measured curvature.

The plurality of series S1 to S6 illustrated in FIG. 8 are not limited to tension values, and may include one of pressure values, stress values, and strain values obtained by measuring the entire or partial upper surface of the user's skull by using the plurality of sensors 211 to 214.

An authorized series Sₐᵤₜ may be a set of tension values obtained by measuring the entire or partial upper surface of the skull of the user, who is an authentication object, with an authority to use the HMD device 1000 by using the plurality of sensors 211 to 214 for registration duration.

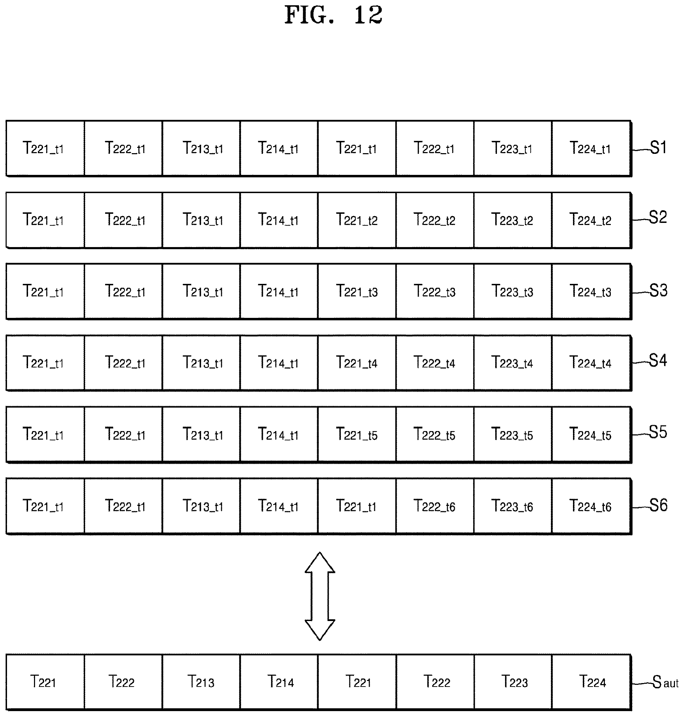

The authorized series Sₐᵤₜ may be stored in the memory 300 (see FIG. 3). In an embodiment, the authorized series Sₐᵤₜ may be a series stored and registered in the memory 300 prior to the authentication duration. In an embodiment, the controller 400 (see FIG. 3) may select one of the plurality of series classified by the plurality of sensors 211 to 214, and compare the selected series with a series of characteristic values registered in advance and stored in the memory 300, thereby authenticating a use authority of the user, which will be described below in a description portion of FIG. 12.

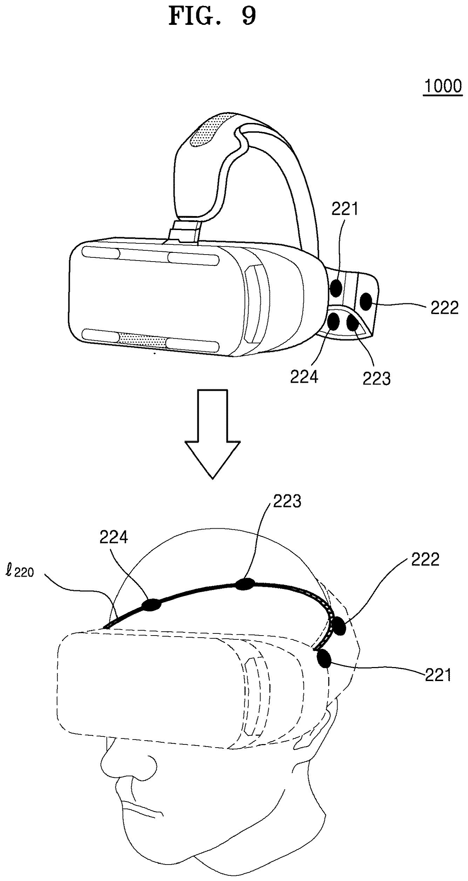

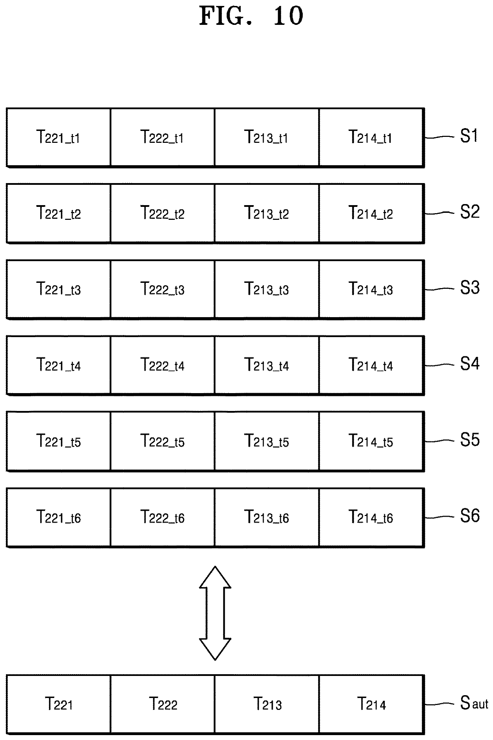

FIG. 9 is a view for explaining a method of measuring, at the HMD device 1000, a characteristic value of shapes of a temporal bone and an occipital bone of a user according to an embodiment, and FIG. 10 is a view illustrating an embodiment of classifying characteristic values obtained by the HMD device 1000 by using the method illustrated in FIG. 9 into a plurality of series.

Referring to FIG. 9, the HMD device 1000 may include the strap band 120 and the plurality of sensors 221 to 224 spaced apart from each other inside the strap band 120. The plurality of sensors 221 to 224 may contact the user's temporal bone and occipital bone with the user's skin tissue disposed therebetween. Specifically, the (2-1)-st sensor 221 may be disposed in one region of the left temporal bone of the user, the (2-2)-nd sensor 222 may be disposed in one region between the left temporal bone and the occipital bone, the (2-3)-rd sensor 223 may be disposed in one region between the occipital bone and the right temporal bone, and the (2-4)-th sensor 224 may be disposed in one region of the right temporal bone. However, the arrangements of the (2-1)-st sensor 221 to the (2-4)-th sensor 224 are not limited to the locations illustrated in FIG. 9.

In an embodiment, the plurality of sensors 221 to 224 may be tension sensors measuring tension (or tensile force) changed by a shape, a size, or a skeletal structure of the user's temporal bone and occipital bone. However, the plurality of sensors 221 to 224 are not limited thereto and may be at least one of pressure sensors, stress sensors, and strain sensors measuring pressure values or stress change values changed by a shape, a size, or a skeletal structure of the user's frontal bone, temporal bone, and occipital bone, but are not limited to the listed examples.

In an embodiment, the plurality of sensors 221 to 224 may measure tension values changed by the shapes of the user's temporal bone and occipital bone for predetermined authentication duration that authenticates a use authority of the user who wears the HMD device 1000, and classify the measured tension values into a plurality of series and list the classified series in a time series. The plurality of sensors 221 to 224 may combine tension values included in each of the plurality of series S1 to S6, measure a curvature of a skeletal structure of the user's temporal bone and occipital bone, and obtain a curvature curve I₂₂₀ for the measured curvature.

Referring to FIG. 10, tension values T_{221_t1} to T_{224_t6} by the shapes of the user's temporal bone and occipital bone measured by using the plurality of sensors 221 to 224 are classified into a plurality of series S1 to S6 and listed. Since a method of obtaining and classifying the plurality of series S1 to S6 is the same as that described in FIG. 8, repeated description is omitted.

A authorized series Sₐᵤₜ may be a set of tension values obtained by measuring a temporal bone and an occipital bone of the user, who is an authentication object, with an authority to use the HMD device 1000 by using the plurality of sensors 211 to 214 for registration duration. Description repeated in FIG. 8 from among the descriptions of the authorized series Sₐᵤₜ is omitted.

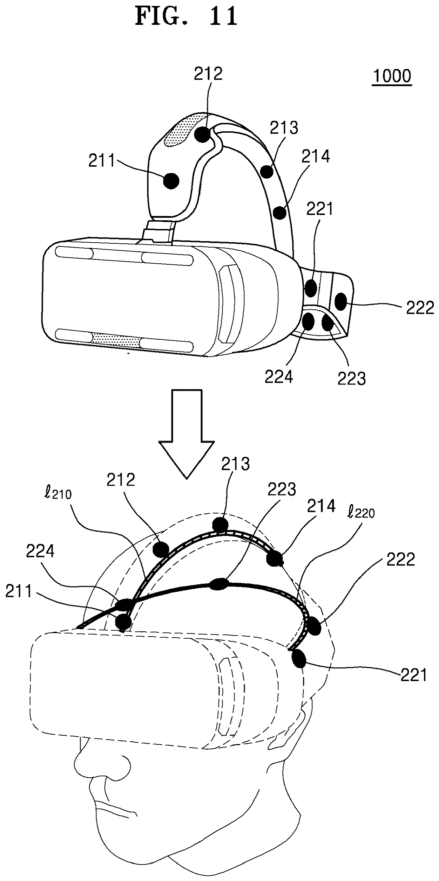

FIG. 11 is a view for explaining a method of measuring, at the HMD device 1000, a characteristic value of the shapes of an upper surface of a skull, a temporal bone, and an occipital bone of a user according to an embodiment, and FIG. 12 is a view illustrating an embodiment of classifying characteristic values obtained by the HMD device 1000 by using the method illustrated in FIG. 11 into a plurality of series.

Referring to FIG. 11, the HMD device 1000 may include the mounting belt 110, the strap band 120, and the plurality of sensors 211 to 224. The (1-1)-st sensor 211 to the (1-4)-th sensor 214 may be spaced apart from each other inside the mounting belt 110. The (2-1)-st sensor 221 to the (2-4)-th sensor 224 may be spaced apart from each other inside the strap band 120. Since the (1-1)-st sensor 211 to the (1-4)-th sensor 214 are the same as the plurality of sensors 211 to 214 illustrated in FIG. 7 and the (2-1)-st sensor 221 to the (2-4)-th sensor 224 are the same as the plurality of sensors 221 to 224 illustrated in FIG. 9, repeated descriptions are omitted.

In an embodiment, the plurality of sensors 211 to 224 may measure tension values changed by the shapes of an upper surface of a skull, a temporal bone, and an occipital bone of a user for predetermined authentication duration that authenticates a use authority of the user who wears the HMD device 1000, and classify the measured tension values into a plurality of series and list the classified series in a time series. The embodiment illustrated in FIG. 11 combines the embodiments illustrated in FIGS. 7 and 9. The HMD device 1000 according to the embodiment may measure tension values changed by the upper surface of a skull, the skeletal shapes of a temporal bone and an occipital bone of the user who wears the HMD device 1000 by using the plurality of sensors 211 to 224, classify the measured tension values in a time series for authentication duration to generate the plurality of series S1 to S6, and combine tension values included in each of the plurality of series S1 to S6 to measure a curvature of skeletal structures of the upper surface of the skull, the temporal bone, and the occipital bone of the user. Also, the HMD device 1000 may obtain a curvature curve I₂₁₀ for the upper surface of the skull, and the curvature curve I₂₂₀ for the temporal bone and the occipital bone.

Referring to FIG. 12, tension values T_{211_t1} to T_{224_t6} by the shapes of the upper surface of the skull, the temporal bone, and the occipital bone of the user measured by using the plurality of sensors 211 to 224 are classified into the plurality of series S1 to S6 and listed. Specifically, in an embodiment illustrated in FIG. 12, the first series S1 may include tension values T_{211_t1} to T_{214_t1} by the shapes of the upper surface of the skull of the user obtained by the plurality of sensors 211 to 214 disposed inside the mounting belt 110, and tension values T_{221_t1} to T_{214_t1} by the shapes of the temporal bone and the occipital bone of the user obtained by the plurality of sensors 221 to 224 disposed inside the strap band 120. The plurality of series S1 to S6 illustrated in FIG. 12 may be a combination of the plurality of series S1 to S6 illustrated in FIGS. 8 and 10. In other words, in the embodiment illustrated in FIG. 12, the HMD device 1000 may measure a curvature of the upper surface of the user's skull and a curvature of the user's temporal bone and occipital bone by combining the tension values T_{221_t1} to T_{224_t6} included in the plurality of series S1 to S6. In an embodiment, the HMD device 1000 may obtain a curvature curve I₂₁₀ corresponding to the shape of the upper surface of the skull of the user who wears the HMD device 1000, and obtain a curvature curve I₂₂₀ corresponding to the user's temporal bone and occipital bone.

The plurality of series S1 to S6 illustrated in FIG. 12 are not limited to tension values and may include characteristic values, for example, one of pressure values, stress values, and strain values obtained by measuring the upper surface of the skull, the temporal bone, and the occipital bone of the user by using the plurality of sensors 211 to 224.

A authorized series Sₐᵤₜ may be a set of tension values obtained by measuring the upper surface of the skull, the temporal bone, and the occipital bone of the user who has an authority to use the HMD device 1000 by using the plurality of sensors 211 to 224 for registration duration. Since the authorized series Sₐᵤₜ is the same as the Sₐᵤₜ illustrated in FIGS. 8 and 10 except a characteristic of including tension values measured for the upper surface of the skull of the user who wears the HMD device 1000 and tension values measured for the user's temporal bone and occipital bone, repeated description is omitted.

FIG. 13 is a flowchart for explaining a method of authenticating a use authority of a user who wears an HMD device via characteristic values of a head shape of the user measured by the HMD device via a plurality of sensors spaced apart from each other according to an embodiment.

In operation S1310, characteristic values of different regions on the skull or face of the user who wears the HMD device are measured by using at least one sensor included in the HMD device.

Referring to FIGS. 7, 9, and 11, the HMD device 1000 may include the plurality of sensors 211 to 214 spaced apart from each other inside the mounting belt 110, and the plurality of sensors 221 to 224 spaced apart from each other inside the strap band 120. In an embodiment, characteristic values may mean tension values measured for the shape of the upper surface of the skull of the user who wears the HMD device 1000 by using the (1-1)-st sensor 211 to the (1-4)-th sensor 214 from among the plurality of sensors 211 to 224, and/or tension values measured for the shape of the user's temporal bone and the occipital bone by using the (2-1)-st sensor 221 to the (2-4)-th sensor 224. However, the characteristic values are not limited thereto and may mean pressure values or stress change values of the user's skull, temporal bone, and occipital bone measured by the plurality of sensors 211 to 224.

In operation S1320, the HMD device classifies the measured characteristic values into at least one series.

In an embodiment, the HMD device may measure characteristic values of the upper surface of the skull of the user, the user's temporal bone, and occipital bone for predetermined authentication duration that authenticates a use authority of the user who wears the HMD device, and classify the measured characteristic values into a plurality of series. The HMD device may classify the characteristic values into a plurality of series depending on measurement time for the authentication duration, and list the plurality of classified series in a time series.

In operation S1330, the HMD device selects one of at least one series. In an embodiment, the HMD device may select one series from among the plurality of series S1 to S6 classified in a time series (see FIGS. 8, 10, and 12). In an embodiment, the selected series may include one of tension values of the upper surface of the user's skull, tension values of the user's temporal bone, and occipital bone, and a combination of these measured by the HMD device at a specific time within the authentication duration.

In operation S1340, the HMD device determines whether the selected series corresponds to a series of characteristic values registered in advance.

In an embodiment, the HMD device may classify characteristic values of a head shape of a user who has an authenticated use authority of the HMD device according to a measured region. For example, the memory may store series in which tension values measured with respect to at least one of the upper surface of the skull, the temporal bone, and the occipital bone of the user who has an authenticated use authority of the HMD device by using tension sensors are classified depending on each of the upper surface of the skull, the temporal bone, and the occipital bone. In another embodiment, the memory may store one series in which tension values measured with respect to at least one of the upper surface of the skull, the temporal bone, and the occipital bone of the user who has an authenticated use authority of the HMD device by using tension sensors are combined.

In an embodiment, the HMD device may determine whether the series selected in operation S1330 corresponds to a series of characteristic values registered in advance. The HMD device may compare characteristic values included in the selected series with the characteristic values of the series registered in advance. In an embodiment, the HMD device may compare tension values included in the selected series with tension values included in the series registered in advance, respectively, and determine whether the difference value is less than a threshold value set in advance. In an embodiment, the HMD device may compare the tension values included in the selected series with the tension values included in the series registered in advance via a Welch t-test.

In operation S1350, when determining that the series selected in operation S1340 corresponds to the series of the characteristic values registered in advance, the HMD device authenticates a use authority of the user who wears the HMD device.

In an embodiment, the HMD device may calculate a difference between a characteristic value, for example, a tension value included in the selected series and a characteristic value, for example, an already registered tension value included in the registered series, and when the calculated difference value is less than a threshold value set in advance, determine that the selected series corresponds to the registered series of the characteristic values.

In an embodiment, when a use authority of the user who wears the HMD device is authenticated, the controller 400 (see FIG. 28) may transmit an electric signal that commands performing an operation of displaying multimedia content or providing virtual reality or augmented reality to a main controller 900 (see FIG. 28).

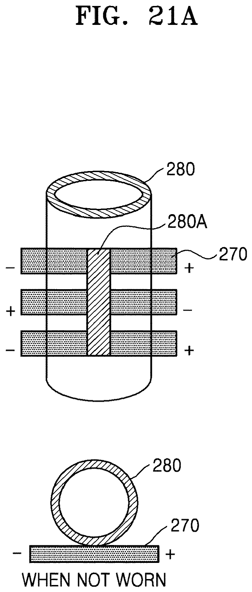

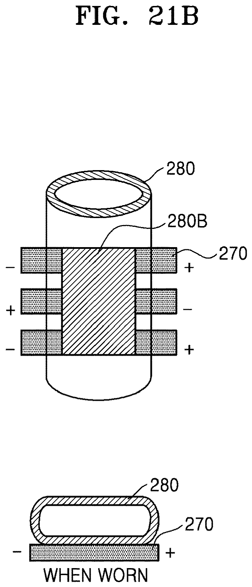

In operation S1360, when determining that the series selected in operation S1340 does not correspond to the series of the characteristic values registered in advance, the HMD device may reject authentication of the user who wears the HMD device. In an embodiment, the HMD device may calculate a difference value between a characteristic value included in the selected series and a characteristic value included in the registered series, and when the calculated difference value is equal to or greater than the threshold value set in advance, determine that the selected series does not correspond to the series of the characteristic values registered in advance. In this case, the HMD device 1000 may be allowed not to perform a separate operation. In an embodiment, the controller 400 (see FIG. 21) may transmit an electric signal that commands turning off power of the HMD device 1000 to the main controller 900 (see FIG. 21).

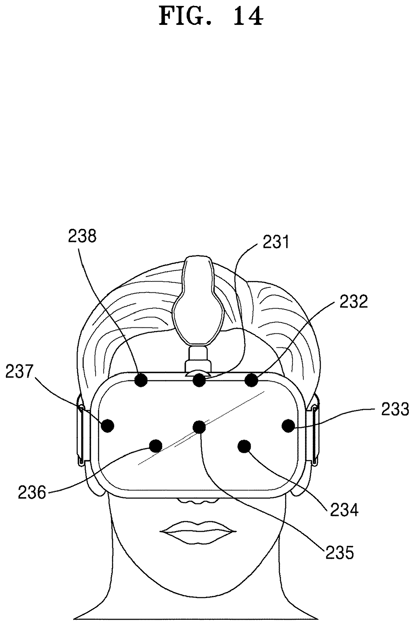

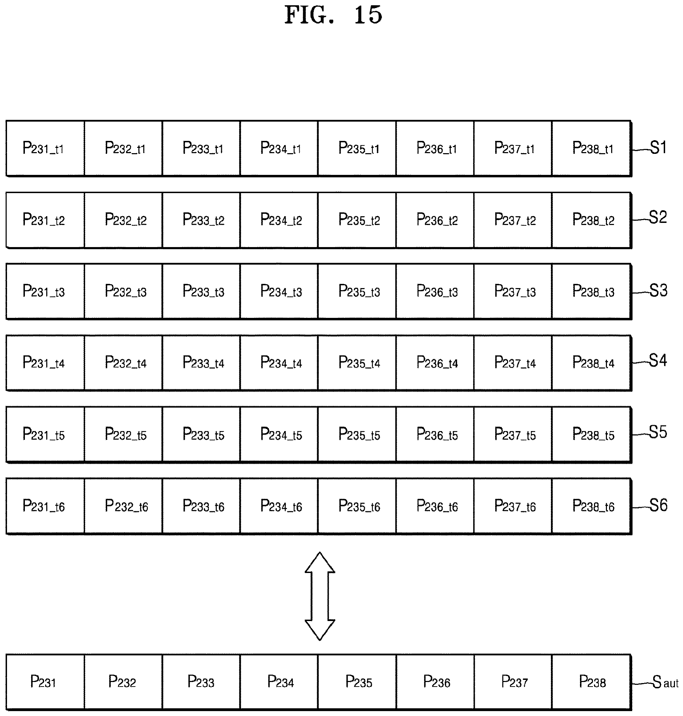

FIG. 14 is a view illustrating locations at which a plurality of sensors 231 to 238 included in the HMD device 1000 and spaced apart from each other contact a user's face according to an embodiment, and FIG. 15 is a view illustrating an embodiment of classifying, at the HMD device 1000, characteristic values of a face shape of a user measured by using the plurality of sensors 231 to 238 illustrated in FIG. 14 into a plurality of series.

Referring to FIG. 14, the HMD device 1000 may include the mask 130 mounted on a portion of a face of a user who wears the HMD device 1000, and the plurality of sensors 231 to 238 spaced apart from each other inside the mask 130. Though the number of the plurality of sensors 231 to 238 is eight in FIG. 14, the number and disposed locations of the plurality of sensors 231 to 238 are not limited to those illustrated.

The plurality of sensors 231 to 238 may contact a portion of the user's face with the user's skin tissue disposed therebetween. The mask 130 may be disposed on a skeleton of the user's the facial portion, for example, at least one of a glabella, a basal bone, an ethmoid bone, a lacrimal bone, a supraorbital foramen, and an infraorbital foramen, and the plurality of sensors 231 to 238 may be disposed on one region of the listed skeletons of the user's the facial portion.

In an embodiment, the plurality of sensors 231 to 238 may be pressure sensors measuring pressure values changed by a skeleton of a facial portion, for example, at least one of a glabella, a basal bone, an ethmoid bone, a lacrimal bone, a supraorbital foramen, and an infraorbital foramen. However, the plurality of sensors 231 to 238 are not limited to the pressure sensors, and may include at least one of tension sensors, stress sensors, and strain sensors. In an embodiment, the plurality of sensors 231 to 238 may measure pressure values changed by the skeleton of the user's the facial portion for predetermined authentication duration that authenticates a use authority of the user who wears the HMD device 1000, and classify the measured pressure values into a plurality of series and list the plurality of series in a time series.

Referring to FIG. 15, pressure values P_{231_t1} to P_{238_t6} by the shape of the skeleton of the user's the facial portion obtained from the plurality of sensors 231 to 238 are classified into a plurality of series S1 to S6 and listed. The plurality of series S1 to S6 may be measured for the authentication duration and obtained in a time series. For example, the first series S1 may be a set of pressure values T_{231_t1} to T_{238_t1} measured at a first time t1 within the authentication duration by using the plurality of sensors 231 to 238. The plurality of sensors 231 to 238 may measure the shape of the skeleton of the facial portion of the user by combining pressure values included in each of the plurality of series S1 to S6. The plurality of series S1 to S6 illustrated in FIG. 15 are not limited to only the pressure values, and may include one of tension values, stress values, strain values, resistance values, and light intensity values that have measured the shape of the skeleton of the facial portion of the user by using the plurality of sensors 231 to 238.

A authorized series Sₐᵤₜ may be a set of pressure values obtained by measuring the shape of the skeleton of the face of the user who is an authentication object having an authority to use the HMD device 1000 by using the plurality of sensors 231 to 238 for registration duration. Since the authorized series Sₐᵤₜ is the same as the authorized series Sₐᵤₜ described with reference to FIGS. 8, 10, and 12 except a characteristic of including a set of pressure values of the shape of the skeleton of the face of the user who is an authentication object, repeated description is omitted.

The HMD 1000 may authenticate a use authority of the user who wears the HMD device 1000 via a characteristic value, for example, a pressure value of the shape of the skeleton of the face of the user measured by using the plurality of sensors 231 to 238. In an embodiment, the controller 400 (see FIG. 3) may classify the pressure values P_{231_t1} to P_{238_t6} measured by using the plurality of sensors 231 to 238 into the plurality of series S1 to S6, select one of the plurality of series S1 to S6, and determine whether the selected series corresponds to the already registered series stored in the memory 300 (see FIG. 3), thereby authenticating the use authority of the user. Since specific description of the method of authenticating the user via a series of characteristic values of the shape of the user's facial skeleton is the same as the description of FIG. 13 except the locations of the plurality of sensors 231 to 238 and regions and kinds of measured characteristic values, repeated description is omitted.

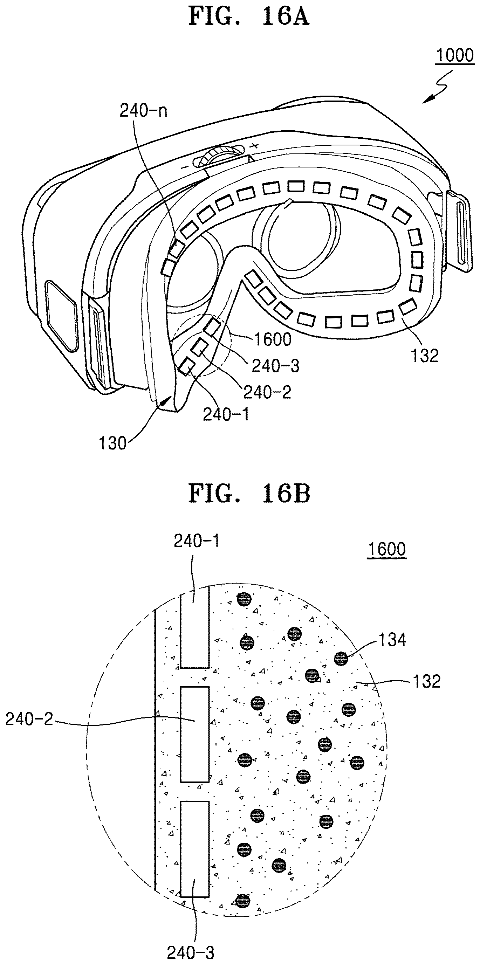

FIG. 16A is a perspective view of an HMD device 1000 including a plurality of conductive sensors 240-1 to 240-n configured to measure a characteristic value regarding a user's facial shape according to an embodiment.

Referring to FIG. 16A, the HMD device 1000 may include a mask 130, and the mask 130 may include a mask pad 132 and the plurality of conductive sensors 240-1 to 240-n. Though FIG. 16A illustrates that the plurality of conductive sensors 240-1 to 240-n protrude from the mask pad 132 and are visible, the illustration is provided for convenience of description. The plurality of conductive sensors 240-1 to 240-n may be arranged inside the mask pad 132.

When a user wears the HMD device 1000, the mask pad 132 may directly contact the user's face. The mask pad 132 may include a conductive foam material. For example, the mask pad 132 may include one of a conductive polymer having conductivity, the conductive polymer including polyacetylene, polyphenylene, and a heterocycle polymer, carbon black, metal powder, and a conductive material adding a metal fiber thereto. However, these materials are provided as examples, and the mask pad 132 is not limited thereto.

The plurality of conductive sensors 240-1 to 240-n may be spaced apart with a predetermined interval along an outer periphery of the mask pad 132 within the mask pad 132. The plurality of conductive sensors 240-1 to 240-n may include a conductive material. For example, though the plurality of conductive sensors 240-1 to 240-n may include a metal material including at least one of Cu, Al, Ni, and Fe, the plurality of conductive sensors 240-1 to 240-n are not limited thereto. The plurality of conductive sensors 240-1 to 240-n may perceive pressure applied thereto, and convert the perceived pressure value to an electric resistance value.

In an embodiment, a current may flow through the mask pad 132. When a user wears the HMD device 1000 and the mask pad 132 is compressed depending on a shape of the user's facial skeleton, pressure is applied to the mask pad 132, and a shape of the mask pad 132 may be transformed by the applied pressure. An amount of a current flowing through the mask pad 132 changes due to the compression of the mask pad 132, and the plurality of conductive sensors 240-1 to 240-n may measure an electric resistance value from a change in the amount of the current.

In an embodiment, when a user wears the HMD device 1000, the plurality of conductive sensors 240-1 to 240-n may perceive a pressure value changed by a contact of the user's face with the mask pad 132, and convert the changed pressure value to an electric resistance value. The HMD device 1000 may store the user's electric resistance value, which serves as an object of user authentication, in a memory 300 (see FIG. 3) of the HMD device 1000. In an embodiment, the HMD device 1000 may authenticate a use authority by comparing an electric resistance value changed by the user's facial skeleton measured by the plurality of conductive sensors 240-1 to 240-n with an authenticated user's resistance value stored in the memory 300 (see FIG. 3).

FIG. 16B is an enlarged cross-sectional view of a first region 1600 of the mask pad 132 of the HMD device 1000 illustrated in FIG. 16A.

Referring to FIG. 16B, the first conductive sensor 240-1, the second conductive sensor 240-2, and the third conductive sensor 240-3 are arranged with a predetermined interval inside the mask pad 132, and current conductive particles 134 may be included in the mask pad 132.

The current conductive particles 134 may include a metal material having current conductivity or a conductive polymer. For example, though the current conductive particles 134 may include a metal including at least one of Cu, Al, Ni, Fe, and Ag, or a polymer coated with a metal, the current conductive particles are not limited thereto.

In an embodiment, when a user wears the HMD device 1000, the current conductive particles 134 may transfer a change in a value of a current flowing through the current conductive particles 134 to the first conductive sensor 240-1, the second conductive sensor 240-2, and the third conductive sensor 240-3 through the mask pad 132. The first conductive sensor 240-1, the second conductive sensor 240-2, and the third conductive sensor 240-3 may measure an electric resistance value corresponding to pressure changed by the user's facial skeleton through the changed current value.

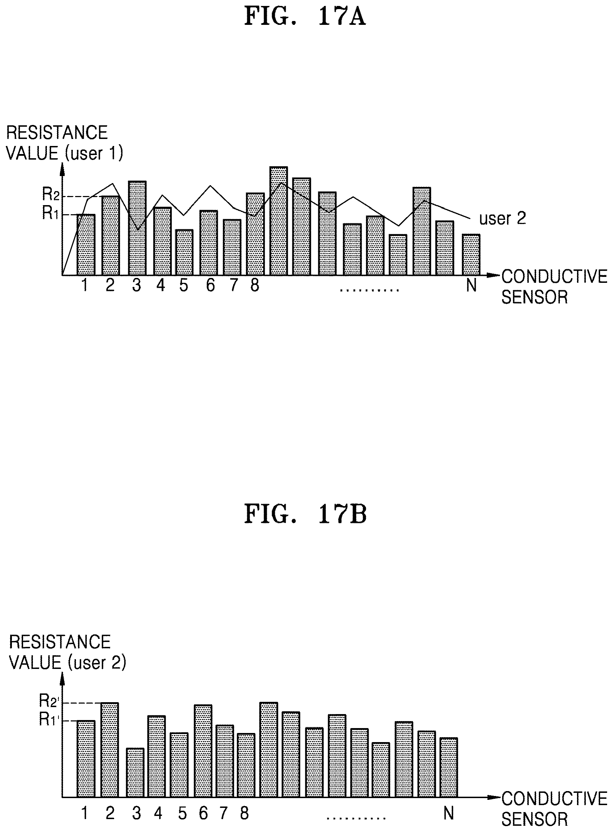

FIGS. 17A and 17B are graphs illustrating characteristic values regarding a shape of a user's facial skeleton measured by a conductive sensor included in the HMD device 1000 according to an embodiment.

Referring to FIG. 17A, in the case where a user 1 wears the HMD device 1000, electric resistance values changed depending on a shape of the user 1's facial skeleton are illustrated as bar graphs corresponding to the plurality of conductive sensors 1 to N. For example, an electric resistance value corresponding to a pressure value changed depending on a shape or a contour of the user 1's facial skeleton measured by the first conductive sensor 1 may be a first resistance value R₁. Likewise, an electric resistance value corresponding to a pressure value changed depending on a shape or a contour of the user 1's facial skeleton measured by the second conductive sensor 2 may be a second resistance value R₂. In an embodiment, electric resistance values measured by the plurality of conductive sensors 1 to N may be values different from each other, and may configure one series and may be stored in the memory 300 (see FIG. 3) of the HMD device 1000.

Referring to FIG. 17B, in the case where a user 2 wears the HMD device 1000, electric resistance values changed depending on a shape of the user 2's facial skeleton may be measured by the plurality of conductive sensors 1 to N and illustrated as bar graphs corresponding to the plurality of conductive sensors 1 to N. An electric resistance value corresponding to a pressure value changed depending on a shape of the user 2's facial skeleton measured by the first conductive sensor 1 may be a resistance value R₁', which may be different from the first resistance value R₁ of FIG. 17A. Likewise, an electric resistance value corresponding to a pressure value changed depending on a shape of the user 2's facial skeleton measured by the second conductive sensor 2 may be a resistance value R₂', which may be different from the second resistance value R₂ of FIG. 17A.

Referring to FIG. 17A again, a line graph of electric resistance values of the user 2 overlaps the bar graph of electric resistance values corresponding to the plurality of conductive sensors 1 to N of the user 1. In an embodiment, the HMD device 1000 may store a series including electric resistance values in the memory 300 (see FIG. 3), the electric resistance values corresponding to the plurality of conductive sensors 1 to N of a user, who is an object of authentication, that is, the user 1. In the case where the user 2 wears the HMD device 1000, the HMD device 1000 may authenticate a use authority by comparing a series including electric resistance values of the user 2 with the series including the electric resistance values of the user 1 stored in the memory 300 in advance. The HMD device 1000 may calculate, for each of the conductive sensors 1 to N, a difference between an electric resistance value changed depending on a shape of a facial skeleton of a user wearing the HMD device 1000 and the electric resistance value of the user 1, who is authenticated, stored in the memory 300 in advance. When the calculated difference is less than a predetermined threshold, the HMD 1000 may authenticate a use authority. When the calculated difference is greater than the predetermined threshold, the HMD 1000 may not authenticate a use authority.

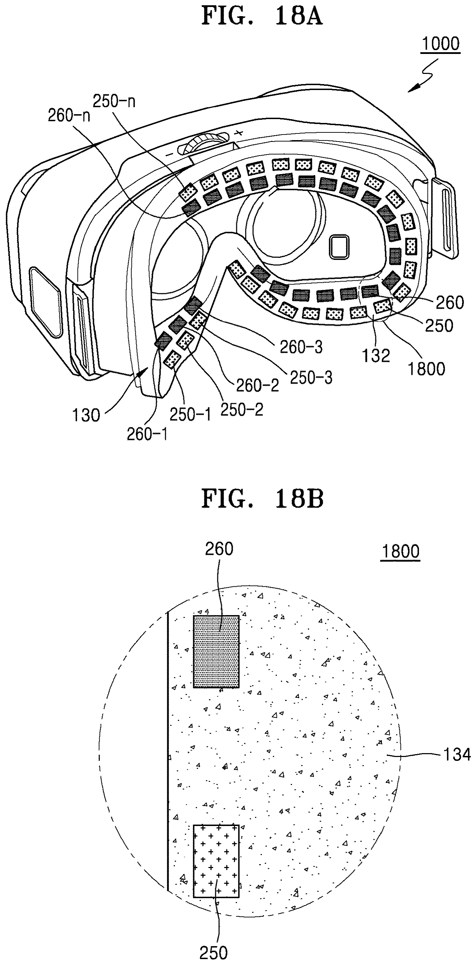

FIG. 18A is a perspective view of the HMD device 1000 including a plurality of lighting elements 250-1 to 250-n and a plurality of photosensitive elements 260-1 to 260-n configured to measure characteristic values regarding a shape of a user's facial skeleton according to an embodiment.

Referring to FIG. 18A, the HMD device 1000 may include a mask 130, and the mask 300 may include a mask pad 134, the plurality of lighting elements 250-1 to 250-n, and the plurality of photosensitive elements 260-1 to 260-n. The plurality of lighting elements 250-1 to 250-n and the plurality of photosensitive elements 260-1 to 260-n may be arranged inside the mask pad 134.

The mask pad 134 may include a translucent foam material configured to absorb light. The mask pad 134 may absorb light emitted from the plurality of lighting elements 250-1 to 250-n.

The plurality of lighting elements 250-1 to 250-n may be spaced apart by a predetermined interval along an outer periphery of the mask pad 134. The plurality of lighting elements 250-1 to 250-n may be arranged as an array along the outer periphery of the mask pad 134 inside the mask pad 134. The plurality of lighting elements 250-1 to 250-n may include a light-emitting diode (LED). However, the lighting elements 250-1 to 250-n are not limited thereto and may include at least one of an organic light-emitting diode (OLED), a semiconductor laser diode (LD), and a solid laser. The plurality of lighting elements 250-1 to 250-n may emit light inside the mask pad 134.

The plurality of photosensitive elements 260-1 to 260-n may be spaced apart by a predetermined interval inside the mask pad 134. The plurality of photosensitive elements 260-1 to 260-n may be surrounded by the array of the lighting elements 250-1 to 250-n along an outer periphery of the mask pad 134. In an embodiment, the plurality of photosensitive elements 260-1 to 260-n may include a photo resistor (PR). The plurality of photosensitive elements 260-1 to 260-n may receive light emitted from the lighting elements 250-1 to 250-n and absorbed by the mask pad 134.

FIG. 18B is an enlarged cross-sectional view of a second region 1800 of the mask pad 134 of the HMD device 1000 illustrated in FIG. 18A.

Referring to FIG. 18B, a lighting element 250 and a photosensitive element 260 may be arranged inside the mask pad 134. The lighting element 250 may be spaced apart from the photosensitive element 260 by a predetermined distance.

In an embodiment illustrated in FIGS. 18A and 18B, in the case where a user wears the HMD device 1000, the user's face contacts the mask pad 134 and thus the mask pad 134 may be compressed. When the mask pad 134 is compressed, intensity of light emitted from the plurality of lighting elements 250-1 to 250-n may be reduced. The HMD device 1000 may authenticate a use authority by comparing light intensity measured by the photosensitive elements 260-1 to 260-n depending on a degree in which the mask pad 134 is compressed according to a user's facial skeleton, with light intensity of an authenticated user stored in the memory 300 (see FIG. 3).

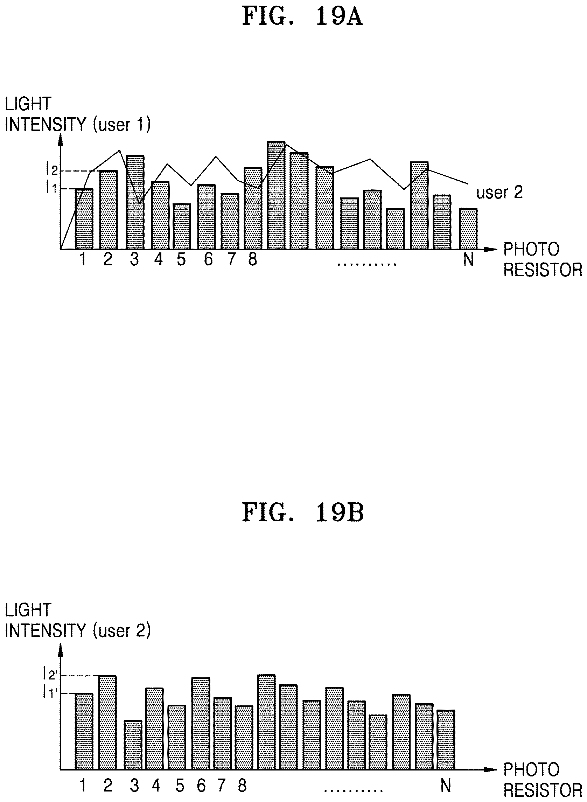

FIGS. 19A and 19B are graphs illustrating characteristic values regarding a shape of a user's facial skeleton measured by a plurality of photosensitive elements included in the HMD device 1000 according to an embodiment.

Referring to FIG. 19A, in the case where a user 1 wears the HMD device 1000, the mask pad 134 (see FIGS. 18A and 18B) is compressed depending on a shape of the user 1's facial skeleton, and intensity of light emitted from a plurality of lighting elements arranged inside the mask pad 134 may change. A plurality of photo resistors 1 to N may measure intensity of light emitted from the plurality of lighting elements, and the measured intensity of light may be illustrated as a bar graph corresponding to the plurality of photo resistors 1 to N. For example, intensity of light changed depending on a shape of the user 1's facial skeleton measured by a first photo resistor 1 may be a first intensity value I₁. Likewise, intensity of light changed depending on a shape of the user 1's facial skeleton measured by a second photo resistor 2 may be a second intensity value I₂. In an embodiment, light intensities measured by the plurality of photo resistors 1 to N may be values different from each other, and may configure one series and may be stored in the memory 300 (see FIG. 3) of the HMD device 1000.

Referring to FIG. 19B, in the case where a user 2 wears the HMD device 1000, light intensities changed depending on a shape of the user 2's facial skeleton may be measured by the plurality of photo resistors 1 to N and illustrated as a bar graph corresponding to the plurality of photo resistors 1 to N. A light intensity changed depending on a shape of the user 2's facial skeleton measured by the first photo resistor 1 is I₁', which may be different from the first intensity value I₁ of FIG. 19A. Likewise, a light intensity changed depending on a shape of the user 2's facial skeleton measured by the second photo resistor 2 is I₂', which may be different from the second intensity value I₂ of FIG. 19A.

FIG. 19A illustrates that a line graph of light intensities changed depending on a shape of the user 2's facial skeleton measured by the plurality of photo resistors 1 to N overlaps a bar graph of measured light intensities depending on a shape of the user 1's facial skeleton. In an embodiment, the HMD device 1000 may store, in the memory 300 (see FIG. 3), a series including light intensities changed depending on a shape of a facial skeleton of a user, who is an object of authentication, that is, the user 1. In the case where the user 2 wears the HMD 1000, the HMD 1000 may authenticate a use authority by comparing a series of the user 2's light intensities with the series of the user 1's light intensities stored in the memory 300 in advance. The HMD device 1000 may calculate, for each of the photo resistors 1 to N, a difference between a light intensity value changed depending on a shape of a facial skeleton of a user wearing the HMD device 1000 and the light intensity value of the user 1, who is authenticated, stored in the memory 300 in advance. When the calculated difference is less than a predetermined threshold, the HMD 1000 may authenticate a use authority. When the calculated difference is greater than the predetermined threshold, the HMD 1000 may not authenticate a use authority.

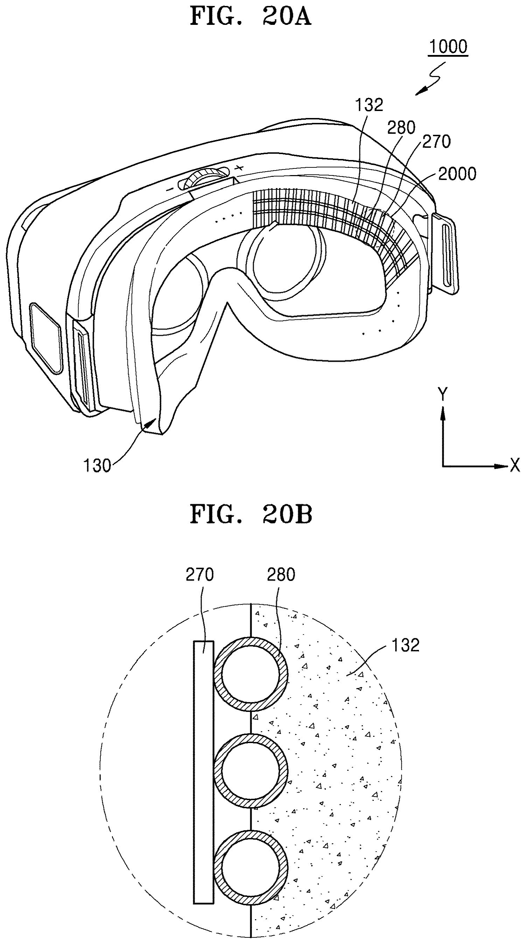

FIG. 20A is a perspective view of the HMD 1000 device including an electrode pad 270 and a conductive rubber tube 280 configured to measure characteristic values regarding a shape of a user's facial skeleton according to an embodiment.

Referring to FIG. 20A, the HMD device 1000 may include a mask 130, and the mask 130 may include the mask pad 132, the electrode pad 270, and the conductive rubber tube 280. Though FIG. 20A illustrates that the electrode pad 270 and the conductive rubber tube 280 protrude from the mask pad 132, the illustration is provided for convenience of description. The electrode pad 270 and the conductive rubber tube 280 may be provided as a plurality of electrode pads and conductive rubber tubes, and arranged inside the mask pad 132. Since the mask pad 132 is the same element as the mask pad 132 illustrated in FIG. 16A, repeated description thereof is omitted.

The electrode pad 270 may extend in a second direction (a Y direction) within the mask pad 132, and may be spaced apart in a first direction (an X direction) by a predetermined interval. The electrode pad 270 is provided as a plurality of electrode pads, and a current may flow through each of the electrode pads 270. The plurality of adjacent electrode pads 270 may be arranged such that the electrodes pads 270 have opposite polarities. Though the electrode pad 270 may include a metal material including at least one of Cu, Al, Ni, Fe, Ag, and Au, the electrode pad 270 is not limited thereto. The conductive rubber tube 280 may extend in the first direction (the X direction) within the mask pad 132, and may be spaced apart in the second direction (the Y direction) by a predetermined interval. The conductive rubber tube 280 may extend on the electrode pads 270 in the first direction (the X direction) across the electrode pads 270. The conductive rubber tube 280 may have a hollow cylindrical shape. The conductive rubber tube 280 may contact the electrode pads 270 on the electrode pads 270. Though the conductive rubber tube 280 may include, for example, a synthetic rubber having conductivity including at least one of nitril butadiene rubber (NBR), epichlorohydrin (ECH) or ethylene propylene (ter) diene monomer (EPDM), the conductive rubber tube 280 is not limited thereto.

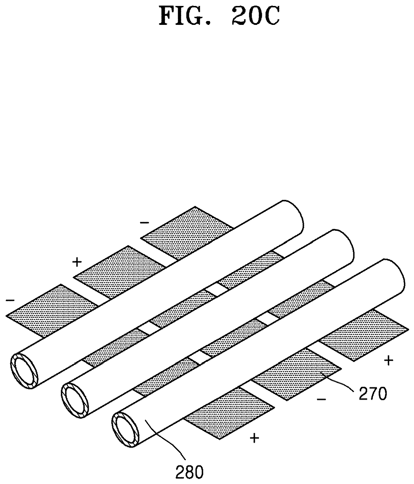

FIG. 20B is an enlarged cross-sectional view of a third region 2000 of the mask pad 132 of the HMD device 1000 illustrated in FIG. 20A, and FIG. 20C is an enlarged perspective view of the electrode pad 270 and the conductive rubber tube 280 illustrated in FIG. 20A.

Referring to FIGS. 20B and 20C, the electrode pad 270 is provided as a plurality of electrode pads, and adjacent electrode pads 270 among the plurality of electrode pads 270 may be arranged such that the adjacent electrode pads 270 have polarities of opposite directions. The conductive rubber tube 280 may have a hollow cylindrical shape of a ring-shaped cross-section. The conductive rubber tube 280 may be arranged on the electrode pads 270 across the electrode pads 270.

The mask pad 132 may be spaced apart from the electrode pads 270 and may contact a portion of the conductive rubber tube 280. In an embodiment, the mask pad 132 may include a foam material having conductivity.

In the case where a user wears the HMD device 1000 illustrated in FIGS. 20A to 20C, pressure is applied to the mask pad 132, and a shape of the conductive rubber tube 280 may be transformed due to the pressure applied to the mask pad 132. When the shape of the conductive rubber tube 280 is transformed, a size of a contact area between the conductive rubber tube 280 and the electrode pad 270 may change, and a current flowing through the electrode pad 270 may change. The HMD device 1000 may measure a change in a resistance value corresponding to a change in a contact area between the conductive rubber tube 280 and the electrode pad 270. The HMD device 1000 may store a resistance value changed depending on a shape of a facial skeleton of a user, who is an object of user authentication, in the memory 300 (see FIG. 3) of the HMD device 1000. The HMD device 1000 may authenticate a use authority by comparing a resistance value corresponding to a size of a contact area between the conductive rubber tube 280 and the electrode pad 270 changed depending on a shape of a facial skeleton of a user wearing the HMD device 1000, with resistance values stored in the memory 300 in advance.

FIGS. 21A and 21B are views for explaining a method of measuring characteristic values regarding a shape of a user's facial skeleton corresponding to a shape change of the conductive rubber tube 280 in the case where a user does not wear the HMD device 1000 and in the case where a user wears the HMD device 1000 according to an embodiment.

Referring to FIG. 21A, in the case where a user does not wear the HMD device 1000, the conductive rubber tube 280 may contact the electrode pad 270 by a first area 280A. In an embodiment, in the case where the electrode pad 270 contacts the conductive rubber tube 280 by the first area 280A, the HMD device 1000 may set a resistance value corresponding to a current flowing through the first area 280A as a default value.

Referring to FIG. 21B, in the case where a user wears the HMD device 1000, the conductive rubber tube 280 is compressed, and may contact the electrode pad 270 by a second area 280B. A compression degree of the conductive rubber tube 280 is different depending on a shape of a facial skeleton of a user wearing the HMD device 1000, and thus a size of the second area 280B may be different depending on a plurality of conductive rubber tubes 280. In an embodiment, in the case where the electrode pad 270 contacts the conductive rubber tube 280 by the second area 280B, the HMD device 1000 may store, in the memory 300 (see FIG. 3), a resistance value corresponding to a current flowing through the second area 280B as a measured resistance value with respect to the default value.

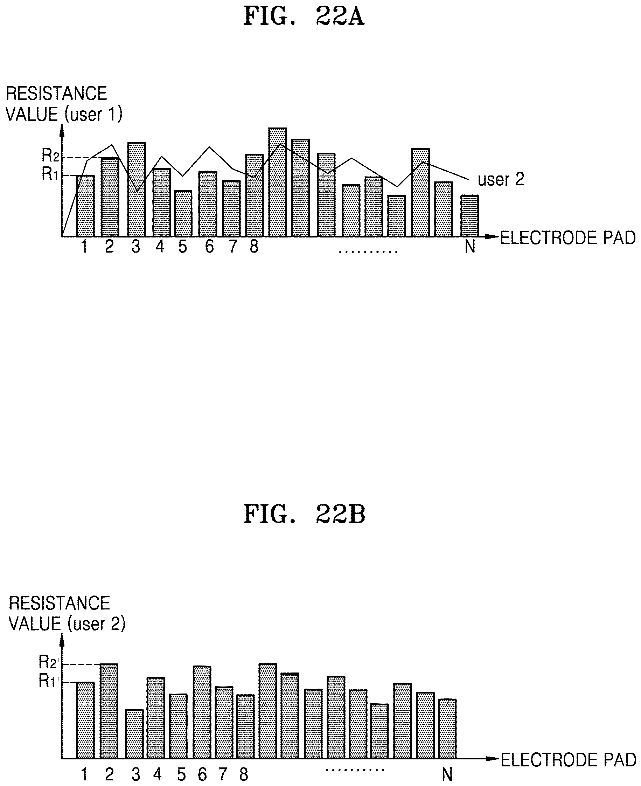

FIGS. 22A and 22B are graphs of characteristic values regarding a user's facial shape detected by the electrode pad 270 included in the HMD device 1000 according to an embodiment.

Referring to FIG. 22A, in the case where a user 1 wears the HMD device 1000, a resistance value corresponding to a change in a size of a contact area between the electrode pad 270 (see FIGS. 21A and 21B) and the conductive rubber tube 280 (see FIGS. 21A and 21B) compressed by a shape of the user 1's facial skeleton is illustrated as a bar graph for each of the plurality of electrode pads 1 to N. For example, a resistance value corresponding to a contact area changed depending on a shape of the user 1's facial skeleton measured by a first electrode pad 1 may be a first resistance value R₁. Likewise, a resistance value corresponding to a contact area changed depending on a shape or a contour of the user 1's facial skeleton measured by a second electrode pad 2 may be a second resistance value R₂. In an embodiment, resistance values measured by the plurality of electrode pads 1 to N may be values different from each other, and may configure one series and may be stored in the memory 300 (see FIG. 3) of the HMD device 1000.

Referring to FIG. 22B, in the case where a user 2 wears the HMD device 1000, a resistance value corresponding to a size of a contact area between the electrode pad 270 (see FIGS. 21A and 21B) and the conductive rubber tube 280 (see FIGS. 21A and 21B) changed depending on a shape of the user 2's facial skeleton is illustrated as a bar graph for each of the plurality of electrode pads 1 to N. A electric resistance value corresponding to a contact area with the conductive rubber tube 280 changed depending on a shape of the user 2's facial skeleton measured by a first electrode pad 1 may be a first resistance value R₁', which may be different from the first resistance value R₁. Likewise, a resistance value corresponding to a contact area with the conductive rubber tube 280 changed depending on a shape of the user 2's facial skeleton measured by a second electrode pad 2 may be a second resistance value R₂', which may be different from the second resistance value R2 of FIG. 22A.

Referring to FIG. 22A, a line graph of resistance values of the user 2 overlaps the bar graph of resistance values corresponding to the plurality of electrode pads 1 to N of the user 1. In an embodiment, the HMD device 1000 may store, in the memory 300 (see FIG. 3), a series including resistance values corresponding to a change in a size of a contact area with the conductive rubber tube 280 respectively measured by the plurality of electrode pads 1 to N of a user, who is an object of authentication, that is, the user 1. In the case where the user 2 wears the HMD 1000, the HMD 1000 may authenticate a use authority by comparing a series of the user 2's resistance values with the series of the user 1's resistance values stored in the memory 300 in advance.

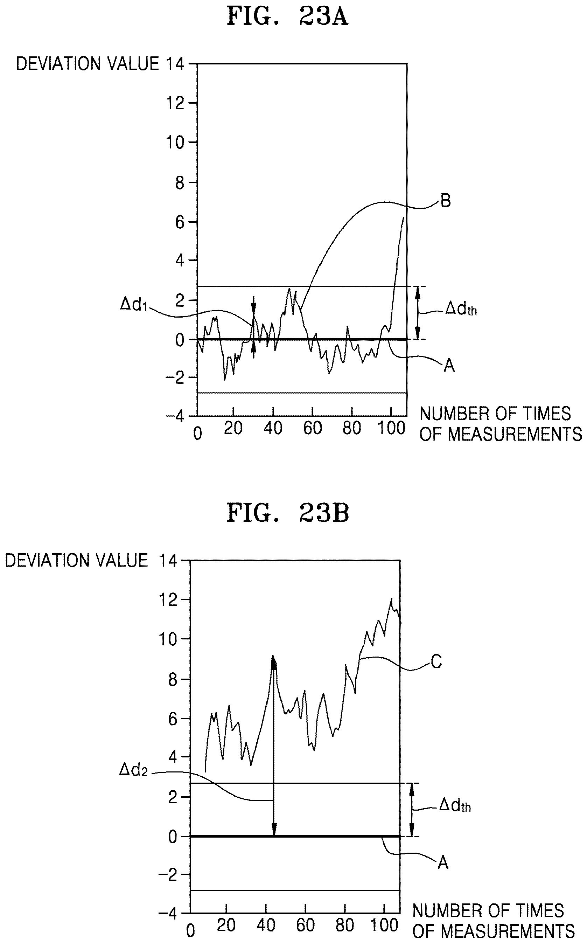

FIGS. 23A and 23B are graphs illustrating deviation between a characteristic value obtained by measuring a characteristic value of a head shape of a user who wears the HMD device 1000 over time and a characteristic value registered in advance according to an embodiment. FIG. 23A is a graph that has measured characteristic values of a head shape of a user who has an authority to use the HMD device 1000, and FIG. 23B is a graph that has measured characteristic values of a head shape of a user who does not have an authority to use the HMD device 1000.

Referring to the graph illustrated in FIG. 23A, an x-axis represents a number of times by which a characteristic value of the head shape of the user who wears the HMD device 1000 is measured by using a plurality of sensors included in the HMD device 1000, and a y-axis represents deviation values between measured characteristic values and characteristic values registered in advance and stored in the memory 300 (see FIG. 3) and used as an authentication object. In an embodiment, a number of times of measurements represented by the x-axis may mean a number of times by which the HMD device 1000 successively measures, over time, characteristic values of a head shape of the user for predetermined authentication duration that authenticates the user who wears the HMD device 1000. The characteristic values registered in advance and stored in the memory 300 may be characteristic values of a head shape of an authenticated user who has an authority to use the HMD device 1000. In an embodiment, the already registered characteristic values may be measured at registration duration different from the authentication duration. The measured characteristic values are values obtained by measuring a head shape of a user who wears the HMD device 1000 over time by using a plurality of sensors for the authentication duration, and may be obtained in a form of a linear sequence.

The graph may be illustrated in parallel to the x-axis, and may include a reference value A having a value of 0 on the y-axis and deviation values B depending on a number of times of measurements. The reference value A may mean deviation between an already registered characteristic value and an already registered characteristic value. Since the already registered characteristic value has the same value over time, deviation has a value of 0 regardless of a number of times of measurements depending on time.

A deviation value B may mean a result value obtained by calculating a difference value between a characteristic value measured for authentication duration and a characteristic value measured and registered for registration duration over time via a predetermined algorithm. In an embodiment, the deviation value B may be a result value obtained by calculating a difference value between a measured characteristic value and an already registered characteristic value via a dynamic time warping algorithm (DTW). The DTW is an algorithm measuring similarity between two linear sequences changing over time, and may measure a distance between two given sequences quantitatively.

In an embodiment illustrated in FIG. 23A, the deviation value B may have a value less than a set threshold value Δdₜₕ during a number of times of measurements within authentication duration. When a difference value Δd₁ between the deviation value B and the reference value A at a specific number of times of measurements within the authentication duration is less than the threshold value Δdₜₕ, the controller 400 (see FIG. 3) may authenticate a use authority of the user who wears the HMD device 1000.

Referring to the graph illustrated in FIG. 23B, the x-axis and the y-axis represent a number of times of measurements and a deviation value, respectively, and the graph may include a deviation value C between a characteristic value of a head shape of a user who does not have an authority to use the HMD device 1000 and a registered characteristic value registered in advance. Since the number of times of measurements and the deviation value denoted by the x-axis and the y-axis, the characteristic value registered in advance, and the deviation value calculation algorithm are the same as those described with reference to FIG. 16A, repeated description is omitted.

The deviation value C may mean a result value obtained by calculating a difference between a characteristic value measured for a head shape of a user who does not have an authority to use the HMD device 1000 for authentication duration and a characteristic value measured and registered for registration duration over time via a predetermined algorithm. In an embodiment, the deviation value C may have a value equal to or greater than the threshold value Δdₜₕ set in advance for the number of times of measurements within the authentication duration. When a difference value Δd₂ between the deviation value C at a specific number of times of measurements within the authentication duration and the reference value A is equal to or greater than the threshold value Δdₜₕ, the controller 400 (see FIG. 3) may reject authentication of a use authority of a user who wears the HMD device 1000.

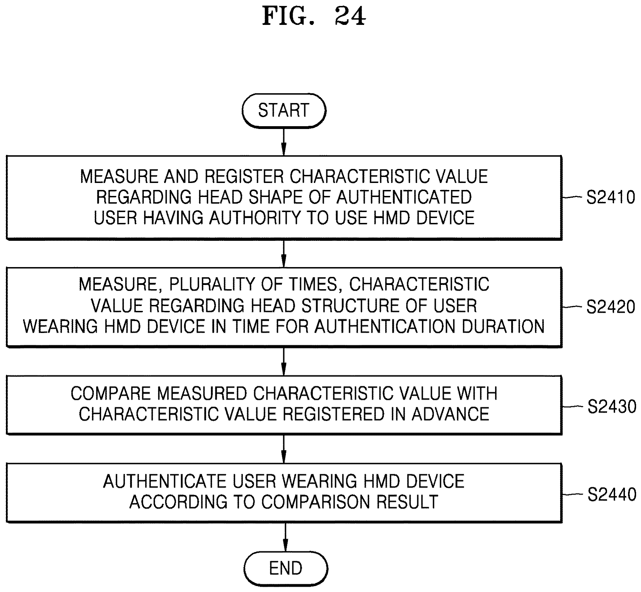

FIG. 24 is a flowchart for explaining a method of measuring a characteristic value of a head shape of a user who wears an HMD device over time, and authenticating a use authority of the user.

In operation S2410, a characteristic value of a head shape of an authenticated user who has an authority to use the HMD device is measured and registered. In an embodiment, the HMD device may measure a characteristic value of a head shape of a user, who is an authentication object, for registration duration.

In operation S2420, a characteristic value of a head structure of a user who wears the HMD device is measured a plurality of numbers of times over time for authentication duration. In an embodiment, the HMD device may successively measure, over time, a characteristic value of a head shape of a user who wears the HMD device for authentication duration. In an embodiment, successively measuring may mean measuring a characteristic value a plurality of numbers of times in a time series.

In operation S2430, the HMD device compares the measured characteristic value with a characteristic value registered in advance.

In an embodiment, the controller 400 (see FIG. 3) may calculate a deviation value between a characteristic value measured for authentication duration by using at least one sensor 200 and a characteristic value registered in advance, over time. In an embodiment, the controller 400 may calculate the deviation value via the DTW.

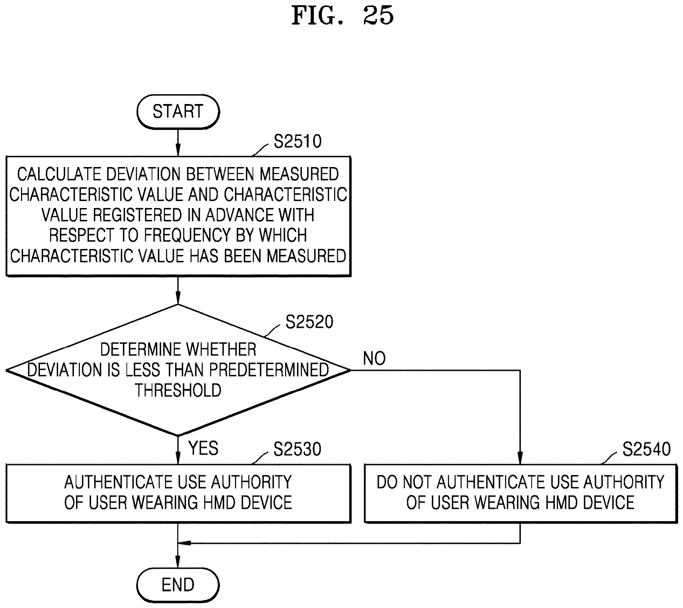

In operation S2440, the HMD device authenticates a use authority of a user who wears the HMD device depending on the comparison result in operation S2430. In an embodiment, when the deviation value calculated in operation S2430 is less than a threshold value set in advance, the HMD device may authenticate a use authority of a user, and when the deviation value is equal to or greater than the threshold value set in advance, reject authentication of the user, which will be described below with reference to FIG. 25.

FIG. 25 is a flowchart for explaining a method of authenticating a use authority of a user by calculating a deviation value between a characteristic value of a head shape of a user who wears an HMD device and a characteristic value registered in advance according to an embodiment.

In operation S2510, the HMD device calculates a deviation value between a measured characteristic value and a characteristic value registered in advance according to a number of times of measurements. In an embodiment, the measured characteristic value is a value obtained by measuring, over time, a head shape of a user who wears the HMD device 1000 a plurality of number of times, and may be obtained in a form of a linear sequence. In an embodiment, the HMD device may calculate a deviation value between a characteristic value measured for authentication duration and a characteristic value registered in advance by using a DTW. The deviation value may be calculated in a form of a sequence over time within the authentication duration.

In operation S2520, the HMD device determines whether the deviation value is less than a threshold value set in advance. Referring to FIGS. 23A and 23B, the calculated deviation value B may be compared with a reference value A for a number of times of measurements within the authentication duration, and whether the calculated deviation value B has a value less than a threshold value Δdₜₕ with respect to both a positive value and a negative value may be determined. In an embodiment illustrated in FIG. 23A, a difference value Δd₁ between the deviation value B and the reference value A is less than the threshold value Δdₜₕ set in advance for all number of times of measurements within the authentication duration. In an embodiment illustrated in FIG. 23B, a number of times of measurements in which a difference value Δd₂ between a deviation value C and the reference value A is equal to or greater than the threshold value Δdₜₕ set in advance within the authentication duration exists.

In operation S2530, when a deviation value is less than the threshold value set in advance for authentication duration, the HMD device authenticates a use authority of a user who wears the HMD device.

Referring to FIG. 23A together, since the controller 400 (see FIG. 3) determines that a difference between the deviation value B and the reference value A is less than the threshold value Δdₜₕ set in advance for all numbers of times of measurements within the authentication duration, the controller 400 may authenticate a use authority of a user who wears the HMD device. In an embodiment, the controller 400 may include a separate module performing an authentication function as in FIG. 28, and when a use authority of the user who wears the HMD device is authenticated, the controller 400 may transmit an electric signal that commands performing an operation of displaying multimedia content or providing virtual reality or augmented reality to the main controller 900 (see FIG. 21).

In operation S2540, when the deviation value is equal to or greater than the threshold value set in advance for the authentication duration, the HMD device 1000 rejects authentication of the user who wears the HMD device 1000. Referring to FIG. 23B together, since the controller 400 has determined that a number of times of measurements in which the difference value Δd₂ between the deviation value C and the reference value A is equal to or greater than the threshold value Δdₜₕ set in advance within the authentication duration exists, the controller 400 may reject authentication of the user who wears the HMD device 1000. In an embodiment, the controller 400 may transmit an electric signal allowing not to perform a separate operation to the main controller 900 (see FIG. 28). Also, the controller 400 may transmit an electric signal that commands turning off power of the HMD device 1000 to the main controller 900.

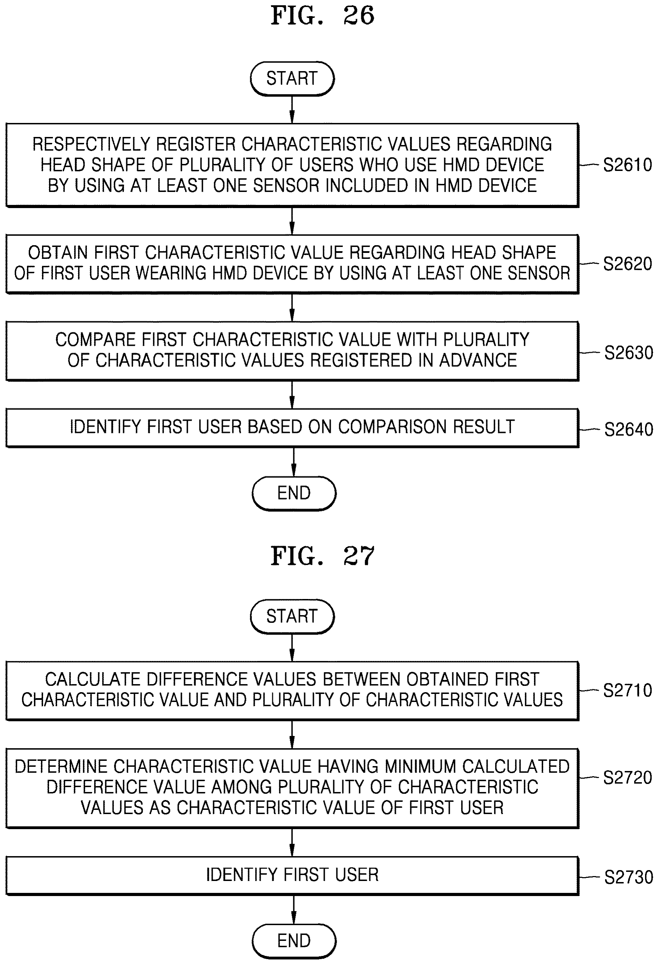

FIG. 26 is a flowchart for explaining a method of identifying a user who wears an HMD device from among a plurality of users who use the HMD device according to an embodiment.

In operation S2610, the HMD device registers characteristic values of head shapes of a plurality of users who use the HMD device. In an embodiment, the HMD device may measure and store at least one of a size, a shape, a skeletal structure, and a curvature of a skeleton of an entire or partial skull of each of the plurality of users who use the HMD device. In another embodiment, the HMD device may measure and store at least one of a size, a shape, a skeletal structure, and a curvature of a skeleton of a temporal bone and an occipital bone of each of the plurality of users who use the HMD device. Also, in another embodiment, the HMD device may measure and store at least one of a size, a shape, a structure, and a curvature of a facial skeleton of each of the plurality of users who use the HMD device.

In an embodiment, the HMD device may classify and store a plurality of characteristic values measured and obtained by each of sensors spaced apart from each other on a series basis, or store a plurality of characteristic values successively obtained over time via a plurality of sensors in a time series.

In operation S2620, the HMD device obtains a first characteristic value of a head shape of a first user who wears the HMD device. In an embodiment, at least one sensor may measure the first characteristic value including at least one of a size, a shape, a skeletal structure, and a curvature of an entire or partial skull, a temporal bone, an occipital bone, and a facial skeleton of the first user.

In operation S2630, the HMD device compares the first characteristic value with each of a plurality of registered characteristic values. In an embodiment, the HMD device may compare a series of measured characteristic values of a head shape of the first user with each of a plurality of series including a plurality of characteristic values stored in the HMD device. In another embodiment, the HMD device may compare characteristic values of a head shape of the first user successively measured over time with a plurality of characteristic values stored in advance, respectively.

In operation S2640, the HMD device identifies the first user based on the comparison result in operation S2630. In an embodiment, the HMD device may determine a characteristic value having a smallest difference value between a plurality of characteristic values and the first characteristic value measured for a head shape of the first user from among the plurality of characteristic values stored in advance as the first characteristic value. In an embodiment, the HMD device may identify the first user via the first characteristic value, and may provide a customized function for the identified first user such as UI setting, customized content providing, etc. stored in response to the first characteristic value.

In the case where a plurality of users use the HMD device, the HMD device described in FIG. 26 may provide user experience UX that has improved use convenience by identifying a user who wears the HMD device and providing a specific function for each user. Particularly, in the case where the HMD device is used in common or used commercially, security of a common HMD device may be improved by identifying a user who uses the HMD device and providing only functions suitable for the user. Also, in the case where the HMD device is used in common by all of family members inside home, use convenience may be improved by implementing functions of blocking content for an adult use in the case where a teenager user wears the HMD device or providing content suitable for an age group or sex.

FIG. 27 is a flowchart for explaining a method of identifying a first user by comparing a characteristic value of a head shape of the first user who wears an HMD device with characteristic values of a plurality of users registered in advance according to an embodiment.

In operation S2710, the HMD device calculates a difference value between an obtained first characteristic value and each of a plurality of characteristic values stored in advance in the HMD device. In an embodiment, the HMD device may calculate a deviation value between the first characteristic value and a plurality of characteristic values by using a DTW. Since the method of calculating the deviation value by using the DTW has been described in FIGS. 23A, 23B, and 25, repeated description is omitted.

In operation S2720, the HMD device determines a characteristic value having a smallest calculated difference value from among the plurality of characteristic values as a characteristic value of the first user. In an embodiment, the HMD device may select the first characteristic value having a smallest deviation value calculated in operation S2710 from among the plurality of characteristic values stored in advance. Also, the HMD device may determine the first characteristic value as a characteristic value of the first user.

In operation S2730, the HMD device may identify the first user who wears the HMD device by determining the first characteristic value as a characteristic value of the first user.

FIG. 28 is a block diagram for explaining components of the HMD device 1000 according to an embodiment.

Referring to FIG. 28, the HMD device 1000 may include the sensor 200, the memory 300, the controller 400, an input/output unit 510, a camera 520, a communication module 600, a display 710, a lens 720, a power management module 800, and the main controller 900.

The sensor 200 may include at least one sensor measuring a characteristic value of a head shape of a user who wears the HMD device 1000. In an embodiment, the sensor 200 may measure geometric parameters including a tension value, a pressure value, a stress value, and a strain value of a skull or face of a user who wears the HMD device 1000. In an embodiment, the sensor 200 may include at least one of a tension sensor, a pressure sensor, a stress sensor, a strain sensor, a conductivity sensor, and a lighting sensor.

In an embodiment, the sensor 200 may include at least one sensor detecting a state of the HMD device 1000 or a state of a neighbor environment of the HMD device 1000. For example, the sensor 200 may include a proximity sensor detecting whether a user approaches the HMD device 1000, a motion/direction sensor detecting an operation (for example, rotation, acceleration, deceleration, vibration, etc.) of the HMD device 1000, an illuminance sensor detecting ambient illuminance, a photosensitive sensor detecting color, spectrum, etc. of light, or a combination of these. Also, the motion/direction sensor may include at least one of an acceleration sensor, a gravity sensor, a geomagnetic sensor, a gyro sensor, an impulse sensor, a global positioning system (GPS), and a compass sensor. For example, a GPS module may receive radio waves from a plurality of GPS satellites on an orbit of the earth, and calculate the location of the HMD device 1000 by using time of arrival from the GPS satellite to the HMD device 1000. Since the sensor 200 is the same as the sensor 200 described in FIG. 3 except the above characteristic, repeated description is omitted.

The memory 300 may store a characteristic value registered in advance of a head structure of an authenticated user having an authority to use the HMD device 1000. In an embodiment, the memory 300 may classify and store characteristic values registered in advance for each series of characteristic values obtained from each of the plurality of sensors 200 spaced apart from each other, or store characteristic values successively obtained from the sensor 200 over time in a time series. In an embodiment, the memory 300 may store a plurality of characteristic values of a head shape of a plurality of users who use the HMD device 1000 measured by using the sensor 200.

The memory 300 may include at least one of an internal memory and an external memory. The internal memory may include, for example, at least one of a volatile memory (for example, dynamic RAM (DRAM), static RAM (SRAM), synchronous dynamic RAM (SDRAM), etc.), a non-volatile memory (for example, one time programmable ROM (OTPROM), programmable ROM (PROM), erasable and programmable ROM (EPROM), electrically erasable and programmable ROM (EEPROM), mask ROM, flash ROM, etc.), a hard disk drive (HDD), and a solid state drive (SSD). The external memory may include, for example, at least one of compact flash (CF), secure digital (SD), micro-SD, mini-SD, extreme digital (xD), and a memory stick. Since the memory 300 is the same as the memory 300 described with reference to FIG. 3, repeated description is omitted.

The controller 400 may compare a characteristic value of a head shape of a user measured by the sensor 200 with a characteristic value registered in advance in the memory 300, thereby authenticating a use authority of the user. In an embodiment, the controller 400 may compare a series of characteristic values of a head shape of a user obtained by the sensor 200 with a series of characteristic values registered in advance, thereby authenticating a use authority of the user. In another embodiment, the controller 400 may compare characteristic values of a head shape of a user successively measured over time by the sensor 200 with characteristic values registered in advance in the memory 300, thereby authenticating a use authority of the user. In an embodiment, the controller 400 may compare a characteristic value of a head shape of a specific user, for example, a first user who wears the HMD device 1000 with a plurality of characteristic values stored in the memory 300, thereby identifying the first user. In an embodiment, the controller 400 may calculate a difference value between a characteristic value of a head shape of the first user and each of the plurality of characteristic values stored in the memory 300, and determine a characteristic value having a smallest calculated difference value from among the plurality of characteristic values as a characteristic value of the first user. The controller 400 may have the same characteristic as that of the controller 400 described with reference to FIG. 3.

The input/output unit 510 includes elements for receiving an input of a user who wears the HMD device 1000, informing a user of information, receiving data from outside, or outputting data to outside. The input/output unit 510 may include at least one speaker 511, at least one microphone 512, at least one button, a connector, a keypad, or a combination of these. However, the embodiment is not limited thereto.

The speaker 511 may output sounds corresponding to various data to the outside of the HMD device 1000 under control of the main controller 900. Also, the speaker 511 may output sounds corresponding to a function performed by the HMD device 1000. A single speaker 511 or a plurality of speakers 511 may be disposed at an appropriate location or locations of the HMD device 1000. In an embodiment, the speaker 511 may be implemented in the form of an earphone.

The microphone 512 may receive voice or sounds from the outside of the HMD device 1000, generate an electric signal based on the received voice or sounds, and output the generated electric signal to the main controller 900. A single microphone 512 or a plurality of microphones 512 may be disposed at an appropriate location or locations of the HMD device 1000. Throughout the specification, a signal may be replaced by data. Also, data may be represented as a data signal.

The communication module 600 may be a wired, wireless, or wired/wireless communication module, and may transmit data from the main controller 900 to an external device via an external communication network or atmosphere in a wired line or wirelessly, or receive data from an electronic device via an external communication network or atmosphere in a wired line or wirelessly, and transfer the received data to the main controller 900. Depending on an embodiment, the communication module 600 may include at least one of a mobile communication module, a wireless LAN module, and a short distance communication module.

The mobile communication module may perform communication with an external device via a mobile communication network by using at least one antenna under control of the main controller 900. The mobile communication module may transmit/receive a radio signal for voice communication, video communication, a short message service (SMS), or a multimedia message service (MMS) to/from a mobile phone, a smartphone, a tablet PC, or other communication devices having a network address such as an Internet protocol (IP).

The wireless LAN module may be connected to the Internet via a wireless access point (AP) in the neighborhood of the wireless AP under control of the main controller 900. The wireless LAN module may support IEEE802.11x of IEEE.

The short distance communication module may perform short distance communication with an external communication device wirelessly under control of the main controller 900. The short distance communication method may include, for example, Bluetooth™, infrared data association (IrDA), Wi-Fi direct communication, near field communication (NFC), or a combination of these.

The camera 520 may include a lens system and an image sensor, and may further include a flash, etc. The camera 520 may convert light input (or captured) via the lens system into an electric image signal, and output the converted image signal to the main controller 900. A user may capture a moving image or a still image by using the camera 520. Also, the camera 520 may be used for receiving an input via a user's motion or gesture.

The lens system may generate an image of an object by allowing light input from the outside to converge. The lens system includes at least one lens, and each lens may be a convex lens, an aspherical lens, etc. The lens system has symmetry with respect to an optical axis passing through the center of the lens system, and the optical axis may be defined as a central axis of the lens system. The image sensor may convert an optical image formed based on external light input via the lens system into an electric image signal. The image sensor may include a plurality of pixel units disposed in an M x M matrix configuration. The pixel unit may include a photodiode and a plurality of transistors. The pixel unit may accumulate charges generated by input light, and a voltage by the accumulated charges may represent illuminance of the input light. In the case of processing one image forming a still image or a moving image, an image signal output from an image sensor includes a set of voltages (that is, pixel values) output from pixel units. The image signal may represent one frame (that is, a still image). Also, a frame may include M x N pixels. For the image sensor, a charge-coupled device (CCD) image sensor, a complementary metal-oxide semiconductor (CMOS) image sensor, etc. may be used.

The display 710 may include a display panel and a controller controlling the display panel. The display panel may be implemented by using various displays such as a liquid crystal display (LCD), an organic light-emitting diode (OLED), an active-matrix organic light-emitting diode (AM-OLED), and a plasma display panel (PDP). The display panel may be implemented so that it is flexible, transparent, or wearable. The display 710 may allow a virtual reality (VR) image or an augmented reality (AR) image to overlap an image of an object captured by the camera 520. Also, in an embodiment, the display 710 may display multimedia content.

The lens 720 may be implemented in a structure detachable from the HMD device 1000. In an embodiment, the lens 720 may not be included in the HMD device 1000, and the HMD device 1000 may include a structure for attaching the lens 720 thereon in substitution for the lens 720. A user may change color of the lens 720 of the HMD device 1000 by replacing the lens 720.

The power management module 800 may supply power to the HMD device 1000 under control of the main controller 900. The power management module 800 may be connected with one or more batteries. Also, the power management module 800 may supply power input from an external power source via a wired cable to the HMD device 1000. In an embodiment, in the case where the controller 400 rejects authentication of a user who wears the HMD device 1000, the power management module 800 may receive an electric signal from the main controller 900 and may not supply power to the HMD device 1000.

The main controller 900 may control an operation of each of components of the HMD device 1000 including the sensor 200, the memory 300, the controller 400, the input/output unit 510, the camera 520, the display 710, the lens 720, and the power management module 800. The main controller 900 may include at least one of a CPU, RAM, ROM, GPU, and a BUS. The CPU, the RAM, the ROM, and the GPU may be connected to each other via the BUS.

The main controller 900 may control transmission/reception operations of an electric signal between the sensor 200, the memory 300, and the controller 400. In an embodiment, when the sensor 200 performs an operation of measuring a characteristic value of a head shape of a user who wears the HMD device 1000, the memory 300 loads a characteristic value registered in advance, and the main controller 900 may transmit the loaded characteristic value and the measured characteristic value to the controller 400. However, the operations are not limited thereto and the sensor 200, the memory 300, and the controller 400 may transmit/receive an electric signal to/from each other even without intervention of the main controller 900.

In an embodiment, in the case where the controller 400 authenticates a use authority of a user who wears the HMD device 1000, the main controller 900 may control the display 710 to display multimedia content or to provide virtual reality or augmented reality. Also, in an embodiment, in the case where the controller 400 rejects authentication of the user who wears the HMD device 1000, the main controller 900 may control the power management module 800 so that power may not be supplied to the HMD device 1000.

In an embodiment, the main controller 900 may perform all functions performed by the controller 400. In this case, the main controller 900 may not include the controller 400.

An embodiment may be also implemented as a form of a recording medium including commands executable by a computer such as a program module executed by a computer. A non-transitory computer-readable recording medium may be an arbitrary available medium that may be accessed by a computer, and includes all of volatile and non-volatile media and separated type and non-separated type media. Also, a non-transitory computer-readable recording medium may include all of a computer storage medium and a communication medium. The computer storage medium includes all of volatile and non-volatile media and separated type and non-separated type media implemented by using an arbitrary method or technology of storing information such as a computer-readable command, a data structure, a program module, or other data. The communication medium typically includes a computer-readable command, a data structure, a program module, or other data of a modulated data signal such as a carrier, or other transmission mechanisms, and includes an arbitrary information transfer medium.

Description of an embodiment is provided exemplarily, and a person of ordinary skill in the art will understand that other specific modifications may be easily made without changing the essential characteristics of the inventive concept. Therefore, the above embodiments should be understood as exemplary and not for purposes of limitation. For example, each component described as a singular form may be implemented in a distributed fashion, and likewise, components described as being distributed may be implemented in the form of a combined form.

The scope of the inventive concept is defined not by the detailed description of the inventive concept but by the appended claims, and all changes and modifications derived from the meaning and scope of the appended claims, and equivalent concept thereof will be construed as being included in the inventive concept.

The invention is defined in the appended claims.

## Claims

1. A head mounted display, HMD, device (1000) comprising:
a plurality of sensors (200) configured to measure (S410) a characteristic value of a head shape of a user who wears the HMD device (1000);
a memory (300) configured to store a characteristic value registered in advance of a head shape of a user who has an authority to use the HMD device (1000); and
a controller (400) configured to authenticate (S420) an authority allowing the user who wears the HMD device (1000) to use the HMD device (1000) based on the measured characteristic value and the characteristic value registered in advance;
wherein the sensors (211-214, 221-224, 231-238) of the plurality of sensors (200) are spaced apart around an entire or partial surface of a skull of the user; and
wherein the sensors (211-214, 221-224, 231-238) of the plurality of sensors (200) are configured to measure the characteristic value indicating a geometric and structural characteristic of the skull of the user who wears the HMD device (1000).

2. The device of claim 1, wherein the sensors (211-214) of the plurality of sensors (200) are disposed inside a mounting belt (110) fixing and supporting the HMD device (1000) on the skull of the user who wears the HMD device (1000) with a space therebetween.

3. The device of claim 1, wherein the sensors (221-224) of the plurality of sensors (200) are spaced apart from each other inside a strap band (220) surrounding the HMD device (1000), a frontal bone, a temporal bone, and an occipital bone of the user who wears the HMD device (1000), and the plurality of pressure sensors are configured to measure pressure values at the frontal bone, the temporal bone, and the occipital bone of the user who wears the HMD device (1000).

4. The device of claim 1, wherein the sensors (231-238) of the plurality of sensors (200) are spaced apart from each other inside a mask (130) covering a portion of a face of the user who wears the HMD device (1000) and the plurality of pressure sensors (200) are configured to measure pressure values according to a shape and a size of a facial skeleton of the user, and to classify characteristic values measured for different regions on a skull of the user who wears the HMD device (1000) or a face of the user according to a measured region and generate the characteristic values as at least one series.

5. The device of claim 4, wherein the controller (400) is configured to select one of the at least one series, and authenticate a use authority of the user who wears the HMD device (1000) by determining whether the selected one of the at least one series corresponds to a series of the characteristic value registered in advance.

6. The device of claim 1, wherein the plurality of sensors (200) is configured to successively measure, over time, the characteristic value of the head shape of the user who wears the HMD device (1000) for an authentication duration.

7. The device of claim 6, wherein the controller (400) is configured to calculate a deviation value between the measured characteristic value and the characteristic value stored in the memory (300) depending on a number of times the plurality of sensors (200) measure the characteristic value of the authentication duration, and when the calculated deviation value is less than a threshold value set in advance, the controller (400) is configured to authenticate a use authority of the user who wears the HMD device (1000).

8. A method of authenticating, at a head mounted display, HMD, device (1000) including a plurality of sensors (200), a user of the HMD device (1000), the method comprising:
measuring a characteristic value of a head shape of the user who wears the HMD device (1000) using the plurality of sensors (200); and
authenticating a use authority of the user who wears the HMD device (1000) based on the measured characteristic value and a characteristic value registered in advance of a head shape of an authenticated user having an authority to use the HMD device;
wherein the sensors (211-214, 221-224, 231-238) of the plurality of sensors (200) are spaced apart around an entire or partial surface of a skull of the user; and
wherein the measuring of the characteristic value comprises measuring the characteristic value indicating a geometric and structural characteristic of the skull of the user who wears the HMD device (1000).

9. The method of claim 8, wherein the measuring of the characteristic value comprises:
measuring a curvature of an entire or partial skull of the user who wears the HMD device (1000).

10. The method of claim 8, wherein the measuring of the characteristic value comprises:
measuring pressure values at a frontal bone, a temporal bone, and an occipital bone of the user who wears the HMD device (1000).

11. The method of claim 8, wherein the measuring of the characteristic value comprises:
classifying characteristic values measured for different regions on a skull of the user who wears the HMD device (1000) or a face of the user into at least one series according to a measured region.

12. The method of claim 11, wherein the authenticating comprises:
selecting one of the at least one series;
determining whether the selected series corresponds to a series of the characteristic value registered in advance; and
authenticating a use authority of the user who wears the HMD device (1000) based on the determining.

13. The method of claim 8, wherein the measuring of the characteristic value comprises:
successively measuring, over time, the characteristic value of the head shape of the user who wears the HMD device (1000) for authentication duration.

14. The method of claim 8, wherein the authenticating comprises:
calculating a deviation value between the measured characteristic value and the characteristic value registered in advance depending on a number of times of measuring the characteristic value during the authentication duration;
comparing the calculated deviation value with a threshold value set in advance; and
when the deviation value is less than the threshold value, authenticating a use authority of the user who wears the HMD device (1000).

15. A non-transitory computer-readable recording medium having recorded thereon a program for executing on a computer the method of claim 8.

## Patentansprüche

1. Kopfmontierte Anzeige-(KMA)-Vorrichtung (1000), umfassend:
eine Vielzahl von Sensoren (200), die dazu konfiguriert sind, um einen charakteristischen Wert einer Kopfform eines die KMA-Vorrichtung (1000) tragenden Nutzers zu messen (S410);
einen Speicher (300), der dazu konfiguriert ist, um einen im Voraus registrierten charakteristischen Wert einer Kopfform eines Nutzers zu speichern, der eine Befugnis hat, die KMA-Vorrichtung (1000) zu nutzen;
und eine Steuerung (400), die dazu konfiguriert ist, um basierend auf dem gemessenen charakteristischen Wert und dem im Voraus registrierten charakteristischen Wert eine Befugnis zu authentifizieren (S420), die es dem die KMA-Vorrichtung (1000) tragenden Nutzer gestattet, die KMA-Vorrichtung (1000) zu nutzen;
wobei die Sensoren (211-214, 221-224, 231-238) der Vielzahl von Sensoren (200) rund um eine gesamte oder teilweise Oberfläche eines Schädels des Nutzers voneinander beabstandet sind;
und wobei die Sensoren (211-214, 221-224, 231-238) der Vielzahl von Sensoren (200) dazu konfiguriert sind, den charakteristischen Wert zu messen, der eine geometrische und strukturelle Eigenschaft des Schädels des die KMA-Vorrichtung (1000) tragenden Nutzers angibt.

2. Vorrichtung nach Anspruch 1, wobei die Sensoren (211-214) der Vielzahl von Sensoren (200) in einem Montagegürtel (110) angeordnet sind, der die KMA-Vorrichtung (1000) am Schädel des die KMA-Vorrichtung (1000) tragenden Nutzers mit einem Abstand dazwischen befestigt und unterstützt.

3. Vorrichtung nach Anspruch 1, wobei die Sensoren (221-224) der Vielzahl von Sensoren (200) in einem die KMA-Vorrichtung (1000), ein Stirnbein, ein Schläfenbein und ein Hinterhauptbein des die KMA-Vorrichtung (1000) tragenden Nutzers umgebenden Gurtband (220) voneinander beabstandet sind und die Vielzahl von Drucksensoren dazu konfiguriert sind, Druckwerte am Stirnbein, am Schläfenbein und am Hinterhauptbein des die KMA-Vorrichtung (1000) tragenden Nutzers zu messen.

4. Vorrichtung nach Anspruch 1, wobei die Sensoren (231-238) der Vielzahl von Sensoren (200) in einer einen Teil eines Gesichts des die KMA-Vorrichtung (1000) tragenden Nutzers abdeckenden Maske (130) voneinander beabstandet sind und die Vielzahl von Drucksensoren (200) dazu konfiguriert sind, um Druckwerte entsprechend einer Form und einer Größe eines Gesichtsskeletts des Nutzers zu messen und charakteristische Werte, die für unterschiedliche Regionen an einem Schädel des die KMA-Vorrichtung (1000) tragenden Nutzers oder einem Gesicht des Nutzers gemessen werden, entsprechend einer gemessenen Region zu klassifizieren und die charakteristischen Werte als mindestens eine Serie zu generieren.

5. Vorrichtung nach Anspruch 4, wobei die Steuerung (400) dazu konfiguriert ist, eine der mindestens einen Serie auszuwählen und eine Nutzungsbefugnis des die KMA-Vorrichtung (1000) tragenden Nutzers durch Bestimmen, ob die auswählte der mindestens einen Serie einer Serie des im Voraus registrierten charakteristischen Werts entspricht, zu authentifizieren.

6. Vorrichtung nach Anspruch 1, wobei die Vielzahl von Sensoren (200) dazu konfiguriert ist, um im Zeitverlauf die charakteristischen Werte der Kopfform des die KMA-Vorrichtung (1000) tragenden Nutzers für eine Authentifizierungsdauer aufeinanderfolgend zu messen.

7. Vorrichtung nach Anspruch 6, wobei die Steuerung (400) dazu konfiguriert ist, um einen Abweichungswert zwischen dem gemessenen charakteristischen Wert und dem im Speicher (300) gespeicherten charakteristischen Wert abhängig von einer Anzahl von Malen, die die Vielzahl von Sensoren (200) den charakteristischen Wert der Authentifizierungsdauer messen, zu berechnen, und, wenn der berechnete Abweichungswert niedriger als ein im Voraus eingestellter Schwellwert ist, die Steuerung (400) dazu konfiguriert ist, eine Nutzungsbefugnis des die KMA-Vorrichtung (1000) tragenden Nutzers zu authentifizieren.

8. Verfahren zum Authentifizieren, an einer kopfmontierten Anzeige-(KMA)-Vorrichtung (1000), enthaltend eine Vielzahl von Sensoren (200), eines Nutzers der KMA-Vorrichtung (1000), wobei das Verfahren umfasst:
Messen eines charakteristischen Werts einer Kopfform des die KMA-Vorrichtung (1000) tragenden Nutzers mithilfe der Vielzahl von Sensoren (200);
und Authentifizieren einer Nutzungsbefugnis des die KMA-Vorrichtung (1000) tragenden Nutzers basierend auf dem gemessenen charakteristischen Wert und einem im Voraus registrierten charakteristischen Wert einer Kopfform eines authentifizierten Nutzers, der eine Befugnis hat, die KMA-Vorrichtung zu nutzen;
wobei die Sensoren (211-214, 221-224, 231-238) der Vielzahl von Sensoren (200) rund um eine gesamte oder teilweise Oberfläche eines Schädels des Nutzers voneinander beabstandet sind;
und wobei das Messen des charakteristischen Werts das Messen des charakteristischen Werts umfasst, der eine geometrische und strukturelle Eigenschaft des Schädels des die KMA-Vorrichtung (1000) tragenden Nutzers angibt.

9. Verfahren nach Anspruch 8, wobei das Messen des charakteristischen Werts umfasst:
Messen einer Krümmung eines gesamten oder teilweisen Schädels des die KMA-Vorrichtung (1000) tragenden Nutzers.

10. Verfahren nach Anspruch 8, wobei das Messen des charakteristischen Werts umfasst:
Messen von Druckwerten an einem Stirnbein, einem Schläfenbein und einem Hinterhauptbein des die KMA-Vorrichtung (1000) tragenden Nutzers.

11. Verfahren nach Anspruch 8, wobei das Messen des charakteristischen Werts umfasst:
Klassifizieren charakteristischer Werte, die für unterschiedliche Regionen an einem Schädel des die KMA-Vorrichtung (1000) tragenden Nutzers oder einem Gesicht des Nutzers gemessen werden in mindestens eine Serie entsprechend einer gemessenen Region.

12. Verfahren nach Anspruch 11, wobei das Authentifizieren umfasst:
Auswählen mindestens eine der mindestens einen Serie;
Bestimmen, ob die ausgewählte Serie einer Serie der in Voraus registrierten charakteristischen Werte entspricht;
und Authentifizieren einer Nutzungsbefugnis des die KMA-Vorrichtung (1000) tragenden Nutzers basierend auf dem Bestimmen.

13. Verfahren nach Anspruch 8, wobei das Messen des charakteristischen Werts umfasst:
aufeinanderfolgendes Messen, im Zeitverlauf, der charakteristischen Werte der Kopfform des die KMA-Vorrichtung (1000) tragenden Nutzers für die Authentifizierungsdauer.

14. Verfahren nach Anspruch 8, wobei das Authentifizieren umfasst: Berechnen eines Abweichungswerts zwischen dem charakteristischen Wert und dem im Voraus registrierten charakteristischen Wert abhängig von einer Anzahl von Malen des Messens des charakteristischen Werts während der Authentifizierungsdauer;
Vergleichen des berechneten Abweichungswerts mit einem im Voraus eingestellten Schwellwert;
und wenn der Abweichungswert geringer ist als der Schwellwert, Authentifizieren einer Nutzungsbefugnis des die KMA-Vorrichtung (1000) tragenden Nutzers.

15. Nicht-transitorisches computerlesbares Aufzeichnungsmedium mit darauf gespeichert einem Programm zum Ausführen auf einem Computer des Verfahrens nach Anspruch 8.

## Revendications

1. Visiocasque, HMD (1000), comprenant :
une pluralité de capteurs (200) configurés pour mesurer (S410) une valeur caractéristique d'une forme de tête d'un utilisateur portant le visiocasque, HMD (1000) ;
une mémoire (300) configurée pour stocker une valeur caractéristique, enregistrée préalablement, d'une forme de tête d'un utilisateur autorisé à utiliser le visiocasque HMD (1000) ; et
un contrôleur (400) configuré pour authentifier (S420) une autorisation permettant à l'utilisateur portant le visiocasque HMD (1000) d'utiliser le visiocasque HMD (1000) sur la base de la valeur caractéristique mesurée et de la valeur caractéristique enregistrée préalablement ;
les capteurs (211-214, 221-224, 231-238) de la pluralité de capteurs (200) étant espacés autour d'une surface intégrale ou partielle du crâne de l'utilisateur ;
et les capteurs (211-214, 221-224, 231-238) de la pluralité de capteurs (200) étant configurés pour mesurer la valeur caractéristique indiquant une caractéristique géométrique et structurelle du crâne de l'utilisateur portant le visiocasque HMD (1000).

2. Visiocasque selon la revendication 1, les capteurs (211-214) de la pluralité de capteurs (200) étant disposés à l'intérieur d'une courroie de montage (110) fixant et supportant le visiocasque (1000) sur le crâne de l'utilisateur portant le visiocasque HMD (1000), avec un espace entre eux.

3. Visiocasque selon la revendication 1, les capteurs (221-224) de la pluralité de capteurs (200) étant espacés les uns des autres à l'intérieur d'une lanière (220) entourant le visiocasque (1000), un os frontal, un os temporal, et un os occipital de l'utilisateur portant le visiocasque HMD (1000), et la pluralité de capteurs de pression étant configurée pour mesurer des valeurs de pression sur l'os frontal, l'os temporal, et l'os occipital de l'utilisateur portant le visiocasque HMD (1000).

4. Visiocasque selon la revendication 1, les capteurs (231-238) de la pluralité de capteurs (200) étant espacés les uns des autres à l'intérieur d'un masque (130) couvrant une partie du visage de l'utilisateur portant le visiocasque HMD (1000), et la pluralité de capteurs de pression (200) étant configurée pour mesurer des valeurs de pression en fonction d'une forme et de dimensions du squelette facial de l'utilisateur, et pour classifier des valeurs caractéristiques mesurées pour différentes régions du crâne de l'utilisateur portant le visiocasque HMD (1000), ou du visage de l'utilisateur en fonction d'une région mesurée, et générer les valeurs caractéristiques comme étant au moins une série.

5. Visiocasque selon la revendication 4, le contrôleur (400) étant configuré pour sélectionner un de l'au moins une série, et authentifier une autorisation d'utilisation de l'utilisateur portant le visiocasque HMD (1000) en déterminant si la série sélectionnée de l'au moins une série correspond à une série de la valeur caractéristique enregistrée préalablement.

6. Visiocasque selon la revendication 1, la pluralité de capteurs (200) étant configurée pour mesurer successivement, à terme, la valeur caractéristique de la forme de la tête de l'utilisateur portant le visiocasque HMD (1000) pour une durée d'authentification.

7. Visiocasque selon la revendication 6, le contrôleur (400) étant configuré pour calculer une valeur d'écart entre la valeur caractéristique mesurée et la valeur caractéristique enregistrée en mémoire (300) en fonction du nombre de fois que la pluralité de capteurs (200) mesure la valeur caractéristique de la durée d'authentification, et lorsque la valeur d'écart calculée est inférieure à une valeur seuil établie préalablement, le contrôleur (400) étant configuré pour authentifier une autorisation d'utilisation de l'utilisateur portant le visiocasque HMD (1000).

8. Méthode d'authentification, à un visiocasque HMD (1000) comprenant une pluralité de capteurs (200), d'un utilisateur du visiocasque HMD (1000), la méthode comprenant :
la mesure d'une valeur caractéristique de la forme de la tête de l'utilisateur portant le visiocasque, HMD (1000) en utilisant la pluralité de capteurs (200) ; et
l'authentification d'une autorisation d'utilisation de l'utilisateur portant le visiocasque HMD (1000) sur la base de la valeur caractéristique mesurée et d'une valeur caractéristique enregistrée préalablement d'une forme de tête d'un utilisateur authentifié possédant l'autorisation d'utiliser le visiocasque, HMD ;
les capteurs (211-214, 221-224, 231-238) de la pluralité de capteurs (200) étant espacés autour d'une surface intégrale ou partielle du crâne de l'utilisateur ; et
la mesure de la valeur caractéristique comprenant la mesure de la valeur caractéristique indiquant une caractéristique géométrique et structurelle du crâne de l'utilisateur portant le visiocasque HMD (1000).

9. Méthode selon la revendication 8, la mesure de la valeur caractéristique comprenant :
la mesure d'une courbure du casque intégral ou partiel de l'utilisateur portant le visiocasque HMD (1000).

10. Méthode selon la revendication 8, la mesure de la valeur caractéristique comprenant :
la mesure de valeurs de pression sur un os frontal, un os temporal, et un os occipital de l'utilisateur portant le visiocasque HMD (1000).

11. Méthode selon la revendication 8, la mesure de la valeur caractéristique comprenant :
la classification de valeurs caractéristiques mesurées pour différentes régions du crâne de l'utilisateur portant le visiocasque HMD (1000), ou du visage de l'utilisateur en fonction d'au moins une série selon une région mesurée.

12. Méthode selon la revendication 11, l'authentification comprenant :
la sélection de l'au moins une série ;
la détermination si la série sélectionnée correspond à une série de la valeur caractéristique enregistrée préalablement ; et
l'authentification d'une autorisation d'utilisation de l'utilisateur portant le visiocasque HMD (1000) sur la base de la détermination.

13. Méthode selon la revendication 8, la mesure de la valeur caractéristique comprenant :
la mesure successive, à terme, de la valeur caractéristique de la forme de la tête de l'utilisateur portant le visiocasque HMD (1000) pour une durée d'authentification.

14. Méthode selon la revendication 8, l'authentification comprenant :
le calcul d'une valeur d'écart entre la valeur caractéristique mesurée et la valeur caractéristique enregistrée préalablement en fonction du nombre de fois que la mesure de la valeur caractéristique est effectuée au cours de la durée d'identification ;
la comparaison de la valeur d'écart calculée et d'une valeur seuil établie préalablement ; et,
lorsque la valeur d'écart est inférieure à la valeur seuil, l'authentification d'une autorisation d'utilisation de l'utilisateur portant le visiocasque HMD (1000).

15. Support d'enregistrement non transitoire lisible par ordinateur, sur lequel a été enregistré un programme pour l'exécution sur un ordinateur de la méthode selon la revendication 8.
